# EUROPEAN PATENT APPLICATION

(11) **EP 4 335 870 A1**
(43) Date of publication of application: **13.03.2024**
(21) Application number: 22194010.9
(22) Date of filing: 06.09.2022
(51) Int. Cl.: C07K 16/10

(54) **PEPTIDE THERAPEUTICS AGAINST SARS-COV-2 SPIKE PROTEIN**

(71) Applicant: NantCell, Inc., Culver City, CA 90232 (US)
(72) Inventor: NELSON, Jay Gardner, Culver City, 90232 (US); BUZKO, Oleksandr, Culver City, 90232 (US); HIGASHIDE, Wendy M, Culver City, 90232 (US); NIAZI, Kayvan, Culver City, 90232 (US); OLSON, Clifford Anders, Culver City, 90232 (US); SOON-SHIONG, Patrick, Culver City, 90232 (US); TANAKA, Shiho, Culver City, 90232 (US)
(74) Representative: AWA Sweden AB

(57) **Abstract**

Proteinaceous therapeutics, such as antibodies and fusion proteins, for preventing, reducing the occurrence of, and/or treating a SARS-CoV-2 infection in a subject are provided herein. The methods provided herein include administering to a subject an antigen binding fragments (Fab fragment) or antibody that binds to the SARS-CoV-2 Spike protein, and/or a DNA construct encoding an anti-Spike Fab fragment.

## Description

### FIELD

The present disclosure relates to proteins that can be administered to subjects for the prevention and/or treatment of COVID-19 and other such virally induced diseases.

### BACKGROUND

Angiotensin-converting enzyme 2 (ACE2) is a regulatory carboxypeptidase of the renin-angiotensin hormone system and functions as regulator of cardiovascular homeostasis. ACE2 is expressed in numerous tissues, including lung alveolar epithelial cells, enterocytes of the small intestine, Leydig cells and Sertoli cells, the renal proximal tubule, and testis. ACE2 is also a receptor for human coronaviruses such as severe acute respiratory syndrome coronavirus (SARS-CoV) and severe acute respiratory syndrome coronavirus 2 (SARS-CoV-2). The widespread expression of ACE2 in various human tissues makes it possible for human coronaviruses to infect effectively and massively.

Currently, there are no known effective treatment options that can reduce or even entirely avoid the pathogenic effects of a coronavirus infection and its sequelae. While several drugs (e.g., remdesivir, hydroxychloroquine, azithromycin, immune suppressive steroids, etc.), have been proposed for treatment of COVID-19, most of the known proposed drugs will not directly affect the virus or prevent its entry into the host cells but will often reduce an overstimulated immune response to the virus. Likewise, there are no known effective vaccine compositions that would help treat or avoid coronavirus disease.

Batlle & al. (2020) Clin. Sci. 134:543-45 hypothesize that a soluble angiotensin converting enzyme 2 (ACE2) extracellular domain, conjugated to a gamma globulin (IgG1) crystallizable fragment (Fc) domain, can serve as a therapeutic against COVID-19 infection.

Batlle & Wysocki describe chimeric ACE2-Fc constructs in International Patent Application Publication No. WO 2018/140456.

Esvelt (2020, "Designing virus-specific sACE2 mimics for competitive inhibition of SARS-CoV-2," available at www.covid19-hpc-consortium.org/projects/5e90e0ca836090007f1ddb39) hypothesizes that ACE2 constructs can be mutated to achieve varieties that have a higher binding affinity to the severe acute respiratory syndrome coronavirus 2 (SARS-CoV-2) Spike protein's receptor-binding domain (Spike-RBD). Hypothetically, such mutants would outcompete natural ACE2 for Spike-RBD, thus blocking the ability of the Spike-RBD to bind ACE2 on human cells.

Jiang & al. (2020) Trends Immunol. 41(5):355-59 report neutralizing monoclonal antibodies (mAbs) against SARS-CoV-2.

### SUMMARY

Disclosed herein are various proteinaceous therapeutics that can be used to treat and/or prevent COVID-19 infection. In certain embodiments, ACE2 decoy peptides and fragments of ACE2 decoy peptides-optionally conjugated to an Fc domain-are disclosed that have a higher affinity for SARS CoV-2 Spike RBD than does human, wild-type ACE2. These ACE2 decoy peptides and fragments thereof can be used to prevent or inhibit viral entry in an individual not actively infected with SARS CoV-2. They can also be used in an individual already infected with SARS-CoV-2 to prevent spread of virus from infected to non-infected cells, thus slowing and containing the disease. Also disclosed herein are nucleotides and nucleotide vectors (including viral vectors) suitable for inducing a subject's own cells to produce these therapeutically effective ACE2 decoy peptides, fragments, and/or chimeras.

Additionally or alternatively, also disclosed herein are various mAbs that specifically bind SARS CoV-2 spike, as well as Fab and scFv fragments of these mAbs. These mAbs and fragments can be used to prevent viral entry in an individual not actively infected with SARS CoV-2. They can also be used in an individual already infected with SARS-CoV-2 to prevent spread of virus from infected to non-infected cells, thus slowing and containing the disease. Also disclosed herein are nucleotides and nucleotide vectors (including viral vectors) suitable for inducing a subject's own cells to produce these therapeutically effective mAbs and/or fragments.

Various objects, features, aspects, and advantages will become more apparent from the following detailed description of preferred embodiments, along with the accompanying drawings.

### BRIEF DESCRIPTION OF THE DRAWINGS

FIG. 1 depicts a crystal structure of the SARS-CoV-2 Spike protein trimer. The trimer's S1 and S2 domains are indicated. Broken lines delineate the portion of the trimer enlarged in FIG. 2.
FIG. 2 depicts a crystal structure of the receptor binding domain (RBD) of the Spike trimer's S1 domain.
FIG. 3 depicts SPR results obtained with N-612-004 mAb. The plot of 3A shows the affinity results against a cleaved Spike peptide, while 3B shows results against the RBD-S1 peptide.
FIG. 4 depicts SPR results obtained with N-612-017 mAb. The plot of 4A shows the affinity results against a cleaved Spike peptide, while 4B shows results against the RBD-S1 peptide.
FIG. 5 depicts a bio-layer interferometry assays illustrating the ability of selected mAbs to block SARS-CoV-2 Spike to ACE2. The line labeled "buffer" shows ACE2 binding to RBD without obstruction. The lines for N-612-002 and N-612-004 substantially overlap across the whole time series. The N-612-017 line runs nearest the horizon.
FIG. 6 depicts a K_{D} determination by SPR for N-612-004 against RBD peptide.
FIG. 7 depicts a K_{D} determination by SPR for N-612-017 against RBD peptide.
FIG. 8 depicts a K_{D} determination by SPR for N-612-002 against SARS-CoV-2 Spike protein construct Sc-S-PP-T4F.
FIG. 9 depicts a K_{D} determination by SPR for N-612-004 against SARS-CoV-2 Spike protein construct Sc-S-PP-T4F.
FIG. 10 depicts a K_{D} determination by SPR for N-612-007 against SARS-CoV-2 Spike protein construct Sc-S-PP-T4F.
FIG. 11 depicts a K_{D} determination by SPR for N-612-014 against SARS-CoV-2 S Spike protein construct Sc-S-PP-T4F.
FIG. 12 depicts a K_{D} determination by SPR for N-612-017 against SARS-CoV-2 Spike protein construct Sc-S-PP-T4F.
FIG. 13 depicts a K_{D} determination by SPR for N-612-017 scFv against RBD peptide.
FIG. 14 depicts a K_{D} determination by SPR for N-612-056 scFv against RBD peptide.
FIG. 15 depicts a K_{D} determination by SPR for N-612-017 scFv against SARS-CoV-2 Spike trimer.
FIG. 16 depicts a K_{D} determination by SPR for N-612-056 scFv against SARS-CoV-2 Spike trimer.
FIG. 17 depicts a conformational structure and a crystal structure of an ACE2-IgG₁Fc.
FIG. 18 illustrates purification of ACE2-IgG₁Fc mutant decoy peptides. FIG. 18A depicts HPLC purification. FIG. 18B shows the purity of the recovered ACE2 IgG₁Fc mutant decoy peptides under reducing and non-reducing conditions.
FIG. 19 depicts the binding affinity of wildtype ACE2 decoy against RBD-SD 1.
FIG. 20 depicts the binding affinity of the T27Y/H34A mutant ACE2 decoy against RBD-SD 1.
FIG. 21 depicts KD determination by SPR for ACE2 wildtype and ACE2 mutant decoy peptides fused to IgG₁Fc.
FIG. 22 illustrates live SARS-CoV-2 neutralization data.
FIG. 23 depicts the stability of wildtype and mutant ACE2 decoy peptides.
FIG. 24 depicts the size of the T27Y/H34A/R273Q triple mutant ACE2 decoy peptide by size exclusion chromatography.
FIG. 25 depicts the size of the T27Y/H34A/R273K triple mutant ACE2 decoy peptide by size exclusion chromatography.
FIG. 26 depicts the size of the T27Y/H34A/R273L triple mutant ACE2 decoy peptide by size exclusion chromatography.
FIG. 27 depicts the size of the T27Y/H34A/H345A triple mutant ACE2 decoy peptide by size exclusion chromatography.
FIG. 28 depicts the size of the T27Y/H34A/H505L triple mutant ACE2 decoy peptide by size exclusion chromatography.
FIG. 29 depicts the size of the T27Y/H34A/H374N triple mutant ACE2 decoy peptide by size exclusion chromatography.
FIG. 30 depicts the size of the T27Y/H34A/H378N triple mutant ACE2 decoy peptide by size exclusion chromatography.
FIG. 31 depicts the ACE2 activity of triple mutant ACE2 decoy peptides.
FIG. 32 depicts the stability of single, double, and triple mutant ACE2 decoy peptides.
FIG. 33 depicts the stability of double and triple mutant ACE2 decoy peptides.
FIG. 34 depicts the stability of various triple mutant ACE2 decoy peptides.
FIG. 35 depicts the stability of avidin-tagged and untagged wildtype ACE2-IgGFc.
FIG. 36 depicts a K_{D} determination by SPR for N-612-017 and N-612-056 against SARS-CoV-2 Spike protein and variants thereof.
FIG. 37 depicts a K_{D} determination by SPR for N-612-004, N-612-017, N-612-017, and N-612-056 against SARS-CoV-2 Spike protein and variants thereof.
FIG. 38 depicts a K_{D} determination by SPR for wildtype ACE2 and T27Y/H34A/H374N ACE2 decoy peptide against SARS-CoV-2 Spike protein and variants thereof.

### DETAILED DESCRIPTION

### I. Definitions

The following definitions refer to the various terms used above and throughout the disclosure.

"Concomitant" or "concomitantly" includes administering an agent (e.g., an antigen binding fragment) in the presence of a further agent *(e.g.,* anIL015 agonist). Concomitant administration in a therapeutic treatment method includes methods in which a first, second, third, or additional agents are co-administered. Concomitant administration also includes methods in which the first or additional agents are administered in the presence of a second or additional agents, wherein the second or additional agents, for example, may have been previously administered. A concomitant therapeutic treatment method may be executed stepwise by different actors. For example, one actor may administer to a subject a first agent (*e.g*., an antigen binding fragment) and a second actor may administer to the subject a second agent *(e.g.,* IL-15 agonist), and the administering steps may be executed at the same time, or nearly the same time. The actor and the subject may be the same entity (e.g., human). Thus, the term embraces both simultaneous administration and substantially simultaneous administration, *i.e*., at about the same time.

As used herein, a mutation position in an ACE2 mutant is designated relative to the corresponding position in SEQ ID NO:135.

"Encoding"-when used to describe a polynucleotide-conveys that when transcription is initiated from the polynucleotide in a wild-type human cell, the transcript produced would be translated into a given protein. That is to say, a polynucleotide "encodes" a polypeptide when the codon triplets of wild-type human tRNA would produce the polypeptide from the polynucleotide according to the ordinary workings of transcription and translation in the wild-type human cell.

"Effective amount" or "therapeutically effective amount" refers to the amount and/or dosage, and/or dosage regime of one or more agent(s) necessary to bring about the desired result *e.g*., an amount sufficient to prevent an infection in a subject, an amount sufficient to reduce the occurrence of an infection in a subject, and/or an amount sufficient to treat an infection in a subject.

"IL-15 agonist" refers to a compound or molecule that binds to and activates the IL-15 receptor ("IL-15Rα"). The type of compound or molecule of the IL-15 agonist is not particularly limited so long as it binds to and activates the IL-15Rα. The IL-15 agonist may be a peptide, protein, small molecule *(e.g.,* a pharmaceutical drug), or oligonucleotide. The peptide or proteins may be a single amino acid sequence or two or more sequences bound via covalent attachments *(e.g.,* disulfide bonds) or non-covalent attachments *(e.g.,* hydrophilic or hydrophobic interactions, hydrogen bonds). In a particular embodiment, the IL-15 agonist is an antibody, modified antibody, chimeric antibody, or a derivative thereof. In a further embodiment, the IL-15 agonist is a superagonist complex, such as an IL-15 derivative bound to an IL-15Rα/IgG1 Fc fusion protein, also known as nogapendekin alfa-imbakicept (NAI). NAI is also known in the literature as N-803, ALT-803, or IL-15_{N72D}:IL-15RαSu/IgG₁. US 9,328,159, which describes NAI, is incorporated herein by reference in its entirety. Clinical trials involving N-803 are described in NCT04385849, which is incorporated herein by reference in its entirety. In some embodiments, the IL-15 agonist is administered alone, and/or as the sole therapeutic agent for the treatment of a disease, disorder, condition, or infection as described herein. In another embodiment, the IL-15 agonist is administered in combination with another compound or molecule, for example in combination with: an antibody or Fab fragment that binds to a Spike protein or modified variant thereof; an ACE decoy peptide or variant thereof; and/or an agent that modulates ACE or ACE2 signaling (e.g., an ACE inhibitor or an angiotensin receptor antagonist).

Binding of the IL-15 agonist to the IL-15Rα induces a signal to downstream elements to activate the IL-15 signaling pathway and activate the cell. Cells expressing IL-15Rα include, but are not limited to, T cells, NK cells, monocytes, macrophages, dendritic cells, keratinocytes, fibroblasts, myocytes, and nerve cells. Guo, et al. Cytokine Growth Factor Rev., 2017. Binding of the IL-15 agonist to the IL-15Rα propagates a signal through the IL-15Rα *(e.g.,* via a conformational change) that initiates the IL-15 signaling pathway to activate an immune response, such as an antiviral response.

"Subject," "individual," and "patient" interchangeably refer to a mammal, preferably a human or a non-human primate, but also domesticated mammals (*e*.*g*., canine or feline), laboratory mammals (*e*.*g*., mouse, rat, rabbit, hamster, guinea pig), and agricultural mammals *(e.g.,* equine, bovine, porcine, ovine). In certain embodiments, the subject can be human (*e.g*., adult male, adult female, adolescent male, adolescent female, male child, female child) under the care of a physician or other health worker. In certain embodiments the subject may not be under the care of a physician or other health worker.

"SARS-CoV-2" refers to severe acute respiratory syndrome coronavirus 2. It is a highly infectious, positive sense, single-stranded RNA virus that causes the respiratory illness known as COVID-19. The sequence of the wildtype virus can be found at www.ncbi.nlm.nih.gov/nuccore/NC_045512.2 (RefSeq NC_045512.2). As used herein, SARS-CoV-2 also includes mutations and variants of the canonical virus, including but not limited to SARS-CoV-2 mutants and variants that can induce COVID-19 symptoms in the global human population. In some embodiments, the SARS-CoV-2 mutants and variants induce any two or more COVID-19 symptoms in at least 10% of the global human population.

COVID-19 is the respiratory illness in humans caused by infection of a subject with SARS-CoV-2, which includes the wildtype virus and mutants and variants thereof. Symptoms of COVID-19 include, but are not limited to headache, confusion, loss of smell and taste, nasal congestion and rhinorrhea, cough, muscle aches and/or pain, sore throat, fever, chills, diarrhea, breathing difficulties, pneumonia, vomiting, dyspnea, hypoxia, respiratory failure, shock, multiorgan dysfunction, and combinations thereof. In some subjects, the symptoms will occur in clusters, e.g., cough, sputum, shortness of breath and fever; or muscle pain, joint pain, headache, and fatigue.

An "agent that modulates at least one of ACE and ACE2 signaling" refers to a compound or molecule that modulates (e.g. increases or decreases) the expression and/or activity of angiotensin converting enzyme (ACE) or ACE2 in particular. In some embodiments, The agent that modulates at least one of ACE or ACE2 signaling may be an ACE inhibitor or a compound that increases ACE2 expression. In some embodiments, the compound may be an ACE inhibitor selected from, but not limited to, the group consisting of Benazepril, Captopril, Fosinopril, Lisinopril, Enalapril, Perindopril, Moexipril, Quinapril, Ramipril, and Trandolapril. In some embodiments, the compound is an angiotensin receptor antagonist selected from, but not limited to, Candesartan, Olmesartan, Valsartan, and Losartan. Additionally or alternatively, the additional agent may be an inhibitor of BDKRB1/2, AGTR1, MAS1, or renin.

"Treat" and "treatment" each refer to a method for reducing, inhibiting, or otherwise ameliorating an infection by administering a therapeutic to a subject in need of treatment. In some embodiments, the subject in need of treatment may include a subject having, diagnosed as having, or suspected to have, an infection. In a particular embodiment, treat or treatment include administering a therapeutic to a subject having, diagnosed as having, or suspected of having COVID-19. Additionally or alternatively, the subject may be infected with SARS-CoV-2, diagnosed with being infected with SARS-CoV-2, or suspected of being infected with SARS-CoV-2. In some embodiments, the subject may be asymptomatic. Treatment includes administration of any of an ACE2 decoy peptide or variant thereof optionally fused to an Fc domain, Fab fragment or antibody that binds to the Spike protein, an IL-15 agonist, IL-2, or an agent that modulates at least one of ACE and ACE2 signaling, alone, or any combination thereof.

### II. ACE2 decoy peptide and variants thereof

The natural cell-surface receptor for SARS CoV-2 is the extracellular domain of ACE2. By mutating the portion of ACE2 that interacts with the SARS-CoV-2 Spike-RBD, one can make ACE2 decoy peptides (or peptide fragments thereof) that bind to the RBD with higher affinity than does the wild-type ACE2 extracellular domain. The modified forms of ACE2 decoy peptide may be changed to preserve at least three domains known to interact with the SARS-CoV Spike glycoprotein. Likewise, ACE2 portions preferably include a leader peptide to allow secretion from a recombinant cell producing ACE2-Fc constructs (discussed herein). Thus, where a truncation is present on the C terminus, the truncation may remove the sequence motif needed for cleavage by ADAM17 and/or the sequence motif needed for cleavage by TMPRSS11D and/or TMPRSS2. These high-affinity ACE2-Fc constructs can effectively "chelate" the virus away from the wild-type ACE2 on the surfaces of a subject's cells.

The ACE2 protein is 805 amino acids long, but in certain preferred embodiments the ACE2 decoy will not include the entire 805 amino acid ACE2 sequence, but will rather consist of a truncation, for example the ACE2 decoy peptide may lack a transmembrane domain. In certain embodiments, the therapeutic protein will contain no more than 700 amino acids, for example no more than 600 amino acids, no more than 500 amino acids, no more than 400 amino acids, no more than 300 amino acids, no more than 200 amino acids, no more than 100 amino acids, or even 50 or fewer amino acids. For example, certain preferred embodiments will consist of amino acid residues 1-740, or 1-615, or 10-400, or 20-100, or 21-614, or 20-50, or 320-360. Wildtype ACE2 is illustrated as SEQ ID NO:135.

Suitable mutations that improve the ACE2 binding affinity for Spike-RBD include mutations to the glutamine at ACE2 position 24 (SEQ ID NO: 136), to the threonine at position 27 (SEQ ID NO: 137), to the aspartic acid at position 30 (SEQ ID NO: 138), to the histidine at position 34 (SEQ ID NO:139), to the glutamic acid at position 35 (SEQ ID NO:140), to the aspartic acid at position 38 (SEQ ID NO:141), to the tyrosine at position 41 (SEQ ID NO:142), to the arginine at position 273 (SEQ ID NO:201), to the histidine at position 345 (SEQ ID NO:202), to the aspartic acid at position 355 (SEQ ID NO:143), to the histidine at position 374 (SEQ ID NO:203), to the histidine at position 378 (SEQ ID NO:204), to the histidine at position 505 (SEQ ID NO:205). Various combinations of these individual point mutations (*e*.*g*., position 24 & position 27, 24 & 30, 24 & 34, 24 & 35, 24 & 38, 24 & 41, 24 & 355, 27 & 30, 27 & 34, 37 & 35, 27 & 38, 27 & 41, 27 & 355, 30 & 34, 30 & 35, 30 & 38, 30 & 41, 30 & 355, 34 & 35, 34 & 38, 34 & 41, 34 & 355, 35 & 38, 35 & 41, 35 & 355, 38 & 41, 38 & 355, 41 & 355, 24 & 27 & 30, 24 & 27 & 34, 24 & 27 & 35, 24 & 27 & 38, 24 & 27 & 41, 24 & 27 & 355, 24 & 30 & 34, 24 & 30 & 35, 24 & 30 & 38, 24 & 30 & 41, 24 & 30 & 355, 24 & 34 & 35, 24 & 34 & 38, 24 & 34 & 41, 24 & 34 & 355, 24 & 35 & 38, 24 & 35 & 41, 24 & 35 & 355, 24 & 38 & 41, 24 & 38 & 355, 24 & 41 & 355, 27 & 30 & 34, 27 & 30 & 35, 27 & 30 & 38, 27 & 30 & 41, 27 & 30 & 355, 27 & 34 & 35, 27 & 34 & 38, 27 & 34 & 41, 27 & 34 & 273, 27 & 34 & 345, 27 & 34 & 355, 27 & 34 & 374, 27 & 34 & 505, 27 & 35 & 38, 27 & 35 & 41, 27 & 35 & 355, 27 & 38 & 41, 27 & 38 & 355, 27 & 41 & 355, 30 & 34 & 35, 30 & 34 & 38, 30 & 34 & 41, 30 & 34 & 355, 30 & 35 & 38, 30 & 35 & 41, 30 & 35 & 355, 30 & 38 & 41, 30 & 38 & 355, 30 & 41 & 355, 34 & 35 & 38, 34 & 35 & 41, 34 & 35 & 355, 34 & 38 & 41, 34 & 38 & 355, 34 & 41 & 355, 35 & 38 & 41, 35 & 38 & 355, 35 & 41 & 355, 38 & 41 & 355, 24 & 27 & 30 & 35, 24 & 27 & 30 & 38, 24 & 27 & 30 & 41, 24 & 27 & 30 & 355, 24 & 30 & 35 & 38, 24 & 30 & 35 & 41, 24 & 30 & 35 & 355, 24 & 35 & 38 & 41, 24 & 35 & 38 & 355, 24 & 38 & 41 & 355, 27 & 30 & 35 & 38, 27 & 30 & 35 & 41, 27 & 30 & 35 & 355, 27 & 35 & 38 & 41, 27 & 35 & 38 & 355, 27 & 38 & 41 & 355, 30 & 35 & 38 & 41, 30 & 35 & 38 & 355, 30 & 38 & 41 & 355, 35 & 38 & 41 & 355, 24 & 27 & 30 & 35 & 38, 24 & 27 & 30 & 35 & 41, 24 & 27 & 30 & 35 & 355, 27 & 30 & 34 & 35 & 41, 27 & 30 & 34 & 35 & 355, 30 & 34 & 35 & 38 & 41, 30 & 34 & 35 & 38 & 355, 30 & 34 & 38 & 41 & 355, 30 & 35 & 38 & 41 & 355, 34 & 35 & 38 & 41 & 355, 24 & 27 & 30 & 34 & 35 & 41, 24 & 27 & 30 & 34 & 35 & 355, 24 & 27 & 34 & 35 & 41 & 355, 24 & 27 & 30 & 34 & 41 & 355, 24 & 27 & 30 & 34 & 35 & 355, 24 & 30 & 34 & 35 & 38 & 41, 24 & 30 & 34 & 35 & 38 & 355, 27 & 30 & 34 & 35 & 38 & 41, 27 & 30 & 34 & 35 & 38 & 355, 27 & 30 & 34 & 35 & 41 & 355, 30 & 34 & 35 & 38 & 41 & 355, 24 & 27 & 30 & 34 & 35 & 38 & 41, 24 & 27 & 30 & 35 & 38 & 355, 24 & 27 & 30 & 34 & 35 & 41 & 355, 24 & 27 & 30 & 34 & 38 & 41 & 355, 24 & 27 & 30 & 35 & 38 & 41 & 355, 24 & 27 & 34 & 35 & 38 & 41 & 355, 24 & 30 & 34 & 35 & 38 & 41 & 355, 27 & 30 & 34 & 35 & 38 & 41 & 355, and 24 & 27 & 30 & 34 & 35 & 38 & 41 & 355) are also useful for improving affinity for RBD, and all such mutations are illustrated in SEQ ID NO:144.

SEQ ID NOs: 136-144, 147-159, and 201-212 are offered only as examples of suitable ACE2 decoy peptides, but many variations on these sequences are also useful for anti-COVID19 therapeutic purposes. For example, polypeptides having at least 70% sequence identity (*i.e*., at least 75% sequence identity, at least 80% sequence identity, at least 85% sequence identity, at least 90% sequence identity, at least 95% sequence identity, at least 96% sequence identity, at least 97% sequence identity, at least 98% sequence identity, or at least 99% sequence identity) to any one of SEQ ID NOs: 136-144, 147-159, and 201-212 are also useful for therapeutic purposes, provided that the molecule retains-broadly-the overall binding site structure and orientation of the individual SEQ ID NOs: 136-144, 147-159, and 201-212 molecules. SEQ ID NOs: 136-144 disclose ACE2 decoy peptide mutants with a potential single mutation within the protein. SEQ ID NOs: 147-159 and 201-205 disclose ACE2 decoy peptide mutants with a specific single mutation within the protein. SEQ ID NOs: 206-212 describe ACE2 decoy peptide mutants with three point mutations (e.g. a triple mutant).

In one embodiment, a double mutant of ACE2 decoy peptide having the mutations T27Y and H34A is contemplated. An essential mechanism for SARS-CoV-1 and -2 infection begins with the viral spike protein binding to the human receptor protein ACE2. A double mutated ACE2 decoy peptide, or peptide of the binding domain, can bind to the viral spike protein with a very high binding affinity and thus outcompete, reduce, or even entirely avoid viral docking. In one embodiment, the α-helix of ACE2 decoy peptide has the mutations T27Y and H34A. This mutated ACE2 decoy peptide may be generated by introducing the T27Y and H34A mutations into the α-helix of ACE2, wherein the α-helix is proximal to the RBD when the RBD and the ACE2 form a binding complex. The mutated α-helix is shown to have a higher binding affinity to the RBD as compared to a wild type α-helix of ACE2. This double mutated ACE2 decoy peptide may be used for treating or preventing COVID-19.

Additionally or alternatively, the ACE2 decoy peptide moiety can be fused to an Fc domain to create a therapeutic protein chimera (also referred to as a hybrid polypeptide, hybrid construct, fusion polypeptide and/or fusion construct). While any Fc domain (e.g., an Fc domain from IgA, IgD, IgE, IgG, IgM, etc.) can be used, in preferred embodiments the chimera will comprise an Fc domain from an IgA or IgG. In additional embodiments, the IgGFc is selected from the group consisting of IgG₁Fc, IgG₂Fc, IgG₃Fc, and IgG₄Fc. For example, the Fc domain can have at least 70% identity (e.g., at least 75%, at least 80%, at least 85%, at least 90%, at least 95%, at least 96%, at least 97%, at least 98%, at least 99%, or even 100% identity) to SEQ ID NO: 145 or 146. In some embodiments, the ACE2 decoy peptide mutants comprising multiple mutations, such as three mutations, for example T27Y and H34A and an additional mutation (e.g. at R273K, R273L, R273Q, H345A, H374N, H378N, and H505L) may be transferred onto an IgA scaffold. In a preferred embodiment, the triple mutation is the T27Y, H34A, and H374N mutation. Fusing the ACE2 moiety to the IgFc domain can be accomplished by incorporating nucleotide sequences encoding the ACE2 mutants and an IgFc scaffold (e.g. an IgAFc scaffold) into a vector, such as an adenoviral vector. The vector is then formulated in a pharmaceutical composition suitable for administration, such as by subcutaneous, intramuscular, or oral delivery. Alternatively, The ACE2 decoy peptide and Fc may be coupled via a linker, *e.g*., a flexible peptide linker. Typically, peptide linkers may have between 5 and 25 amino acids and all known flexible linkers are deemed appropriate for use herein. Particularly suitable linkers include a run of glycines interspersed with serines (e.g., GGGS). Additionally or alternatively, the ACE2-Fc hybrid construct may be immobilized on a carrier. All manners of modifications to permit immobilization are suitable, *e*.*g*., biotinylation, cellulose binding domain, etc. A detectable label may also be added to the ACE2-Fc hybrid construct to enable *in situ* detection and/or quantification in a quantitative assay. Suitable labels include luminescent labels, radioisotope labels, enzymatic labels, etc.

### III. Antigen binding fragments and antibodies

Antigen binding fragments (Fab fragments) and antibodies that bind to the SARS-CoV-2 Spike protein-and *particularly* antibodies that bind to the Spike RBD-can interfere with SARS-CoV-2 entry into a host cell. The antibodies and Fab fragments disclosed herein can bind SARS-CoV-2 Spike, including several that can bind the Spike RBD.

To illustrate the anti-Spike antibodies and Fab fragments disclosed herein, exemplary Fab fragments are described having a heavy chain variable region (V_{H}) with a structure A-V_{H} CDR1-B-V_{H} CDR2-C-V_{H} CDR3-D. These exemplary anti-Spike Fab fragments also have a light chain variable region (V_{L}) with a structure SEQ ID NO:131-V_{L} CDR3-E. Amino acid sequences for the V_{H} & V_{L} of various such Fab fragments are set forth in Table 1 below. The binding affinities of certain of these Fab fragments have been confirmed by surface plasmon resonance (SPR).

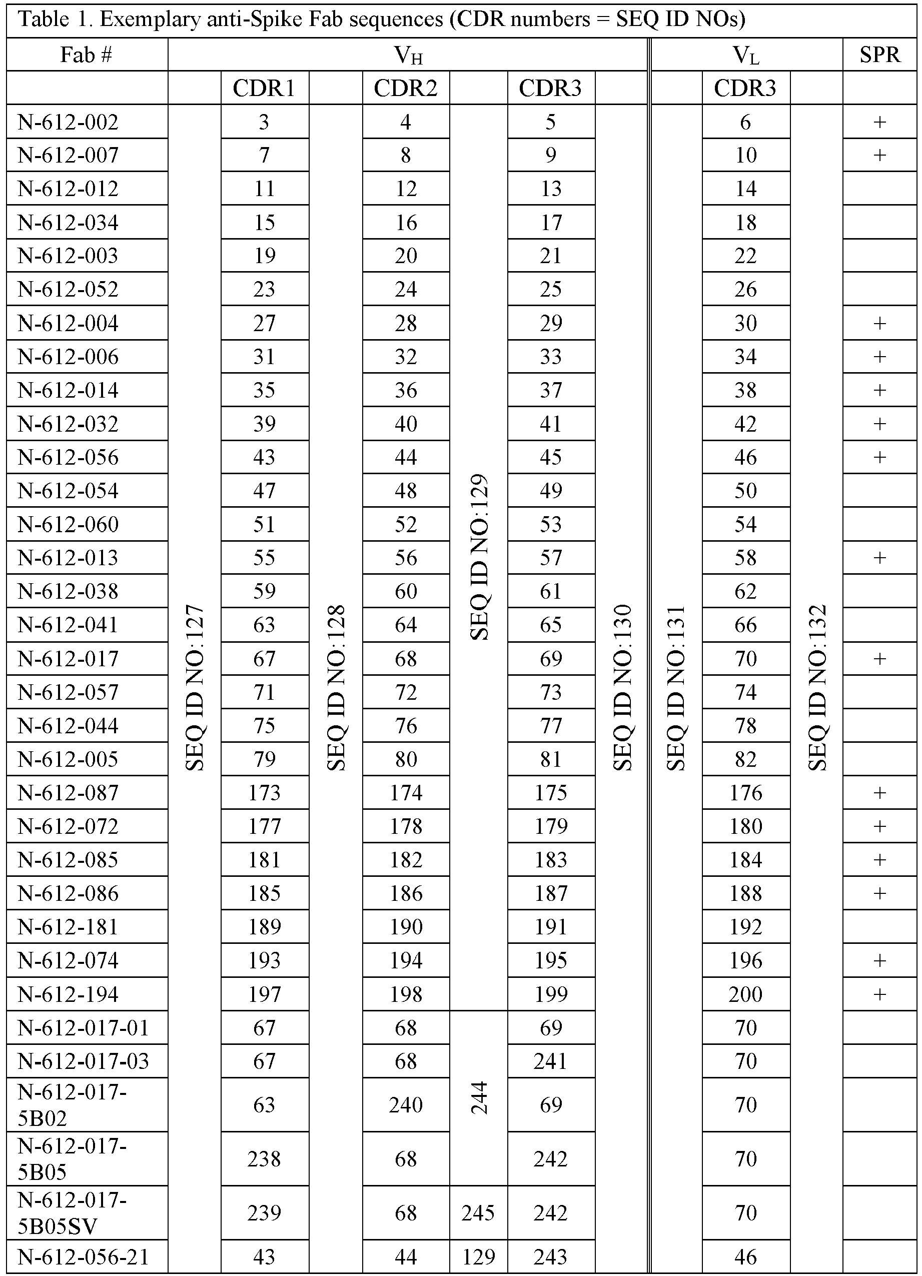

Certain of the anti-Spike Fab fragments bind specifically to the Spike RBD. Amino acid sequences for the V_{H} & V_{L} of various such Fab fragments are set forth in Table 2 below. The binding affinities of certain of these Fab fragments for the Spike protein trimer and/or for an RBD peptide have been confirmed by surface plasmon resonance (SPR).

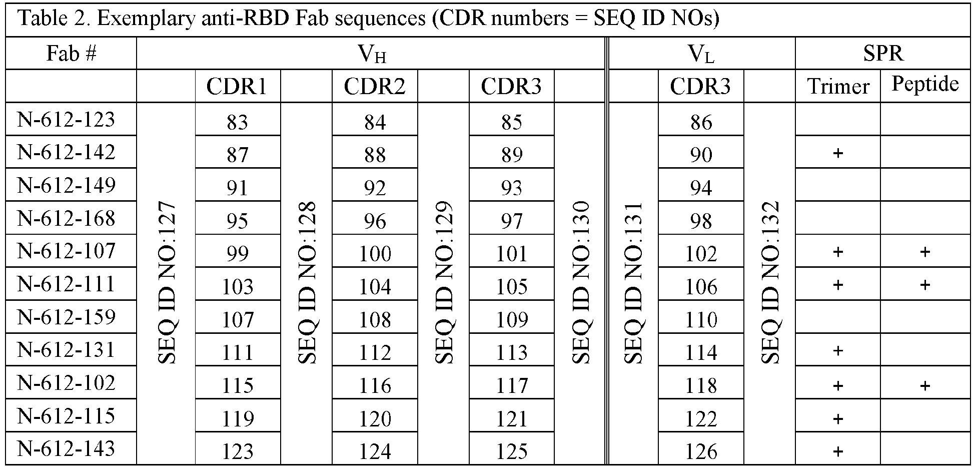

Notwithstanding the particular embodiments set out in Tables 1 and 2, the skilled person understands that the affinity of an antibody or Fab fragment is determined by the complementarity determining regions (CDRs), and that a great deal of change and adjustment can be made to the non-CDR portions of the variable domains without materially altering the usefulness of these antibodies or Fab fragments in neutralizing SARS-CoV-2 infectivity. Therefore, many sequences beyond those exemplified above are equally useful for treating COVID-19 disease. Particularly suitable sequences will retain at least 70% identity (e.g., at least 75%, at least 80%, at least 85%, at least 90%, at least 95%, at least 96%, at least 97%, at least 98%, or at least 99%) to SEQ ID NO:127, at least 70% identity (e.g., at least 75%, at least 80%, at least 85%, at least 90%, at least 95%, at least 96%, at least 97%, at least 98%, or at least 99%) to SEQ ID NO:128, at least 70% identity (e.g., at least 75%, at least 80%, at least 85%, at least 90%, at least 95%, at least 96%, at least 97%, at least 98%, or at least 99%) to SEQ ID NO:129, 244, or 245 at least 70% identity (e.g., at least 75%, at least 80%, at least 85%, at least 90%, at least 95%, at least 96%, at least 97%, at least 98%, or at least 99%) to SEQ ID NO:130, and at least 70% identity (e.g., at least 75%, at least 80%, at least 85%, at least 90%, at least 95%, at least 96%, at least 97%, at least 98%, or at least 99%) to SEQ ID NO:132. Similarly, particular suitable sequences will retain at least 70% identity (e.g., at least 75%, at least 80%, at least 85%, at least 90%, at least 95%, at least 96%, at least 97%, at least 98%, or at least 99%) to SEQ ID NO:131, except that such sequences will retain 100% identity to the light chain CDR1 and CDR2 within the SEQ ID NO:131 sequence.

In some embodiments, Fab fragments and antibodies that bind to the Spike protein (e.g., the Spike RBD) may additionally or alternatively bind to a modified and/or mutated Spike protein. The modified and/or mutated Spike protein may be found on Sars-Cov-2 variants. The modified Spike protein may differ from wild-type or naturally occurring Spike protein by containing point mutations, insertions, deletions, and/or inversions. In an embodiment, the one or more point mutations are selected from the group consisting S13I, L18F, T19R, T20N, P26S, A67V, ΔH69, ΔV70, G75V, T76I, D80A, T95I, D138Y, ΔL141, ΔG142, G142D, ΔV143, ΔY144, ΔY145, W152C, E154K, E156G, ΔF157, ΔR158, R190S, N211I, ΔL212, R214ins, D215G, ΔL241, ΔL242, L242H, ΔA243, ΔL244, R246N, ΔS247, ΔY248, ΔL249, ΔT250, ΔP251, ΔG252, ΔD253, G339D, S371L, S373P, S375F, K417N, K417T, N440K, G446S, S447N, L452Q, L452R, T478K, E484A, E484K, E484Q, F490S, Q493R, G496S, Q498R, N501Y, Y505H, T547K, A570D, D614G, H655Y, N679K, P681H, P681R, A701V, T716I, D796Y, N858K, T859N, D950N, Q954H, S982A, N969K, L981F, Q1071H, E1095K, H1101Y, D1118H, T1027I, V1176F and combinations thereof. Particular multiple mutations include, but are not limited to those listed in Table 3 below:

| **Table 3:** | | | |
|---|---|---|---|
| **WHO Label** | **Pango lineage** | **Alternate Name** | **Mutations** |
| Alpha | B.1.1.7 | UK variant | ΔH69, ΔV70, ΔY144, N501Y, A570D, P681H, T716I, S982A, D1118H |
| (Beta or South Africa variant) | B.1.351 (501.V2) | South Africa variant | D80A, D215G, ΔL241, ΔL242, L242H, ΔA243, K417N, E484K, N501Y, D614G, A701V |
| Gamma | B.1.1.248 (P.1) | Brazil Variant | L18F, T20N, P26S, D138Y, R190S, K417T, E484K, N501Y, D614G, H655Y, T10271, V1176F |
| Delta | B.617.2 | | T19R, G142D, E156G, ΔF157, ΔR158, L452R, T478K, D614G, P681R, D950N |
| Epsilon | B.1.427/B.1.429 | CAL.20C or California variant | S13I, W152C, L452R, D614G |
| Zeta, Eta, or Iota | P.2 | | E484K, D614G, V1176F |
| Theta | P.3 | | ΔL141, ΔG142, ΔV143, ΔA243, ΔL244, E484K, N501Y, D614G, P681H, E1095K, H1101Y, V1176F |
| Kappa | B.1.617.1 | | G142D, E154K, L452R, E484Q, D614G, P681R, Q1071H |
| Lambda | C.37 | | G75V, T76I, R246N, ΔS247, ΔY248, ΔL249, ΔT250, ΔP251, ΔG252, ΔD253, L452Q, F490S, D614G, T859N |
| Omicron | BA.1 | | A67V, ΔH69, ΔV70, T95I, G142D, ΔV143, ΔY144, ΔY145, N211I, ΔL212, R214ins, G339D, S371L, S373P, S375F, K417N, N440K, G446S, S447N, T478K, E484A, Q493R, G496S, Q498R, N501Y, Y505H, T547K, D614G, H655Y, N679K, P681H, N764K, D796Y, N858K, Q954H, N969K, L981F |

### IV. Polynucleotides and vectors

Contemporary molecular biologists know how to make nucleotides that express the proteins described herein, and how to express such nucleotides in cells to obtain the relevant proteins. Further embodiments provided herein include polynucleotides that encode a polypeptide comprising an ACE2 decoy peptide, wherein the ACE2 decoy peptide is a polypeptide of SEQ ID NO:135 or a variant thereof. For example, a polynucleotide encoding wild-type human ACE2 is presented herein as SEQ ID NO:168. The ordinary molecular biologist knows how to alter the sequence of SEQ ID NO:168 to encode SEQ ID NOs: 136-144, 147-159, and 201-212 and appropriate variants thereof (e.g., variants having at least 70% identity (e.g., at least 75%, at least 80%, at least 85%, at least 90%, at least 95%, at least 96%, at least 97%, at least 98%, or at least 99% to any one of SEQ ID NOs: 136-144). Non-limiting examples of polynucleotides encoding SEQ ID NOs: 206-212 are provided herein as SEQ ID NOs: 213-219, respectively. Non-limiting examples of polynucleotides encoding suitable Fc domains are provided herein as SEQ ID NOs: 169 & 170. In a particular embodiment, the polynucleotide encodes an ACE2 decoy polypeptide wherein the threonine at position 27 is replaced with tyrosine (T27Y), the histidine at position 34 is replaced with alanine (H34A), and the histidine at position 374 is replaced with asparagine (H374N). In a particular embodiment, the polynucleotide is characterized by SEQ ID NO: 216. In a further embodiment, the ACE2 decoy peptide or variant thereof is fused or otherwise linked to an IgFc to form a fusion polypeptide. In a particular embodiment, the IgFc is selected from the group consisting of IgAFc, IgDFc, IgEFc, IgGFc, and IgMFc. Where the IgFc is an IgGFc, the Fc may be an IgG₁Fc, IgG₂Fc, IgG₃Fc, or IgG₄Fc.

Similarly, examples of suitable polynucleotides encoding Fab heavy and light chains are presented herein as SEQ ID NOs: 171 & 172. The ordinary molecular biologist knows how to alter the sequences of SEQ ID NOs: 171 & 172 to encode Fab fragments with the relevant CDRs of the anti-Spike and anti-RBD antibodies disclosed herein. Similarly, the ordinary molecular biologist knows how to alter SEQ ID NOs: 171 & 172 further to encode an Fc domain or other such extended structure, if the user wishes to express these Fab fragments in the context of whole immunoglobulins. In an embodiment, the polynucleotide encodes a Fab fragment comprising a heavy variable (V_{H}) chain having at least 85% sequence identity to each of SEQ ID NOs: 127-130 and a light variable (V_{L}) chain having at least 85% identity to each of SEQ ID NOs: 131 & 132, wherein each of the V_{H} chain and the V_{L} chain have three complementarity determining regions (CDRs). Examples of CDR combinations for the V_{H} chain include SEQ ID NOs: 3-5; 7-9; 11-13; 15-17; 19-21; 23-25; 27-29; 31-33; 35-37; 39-41; 43-45; 47-49;51-53; 55-57; 59-61; 63-65; 67-69; 71-73; 75-77; 79-81; 83-85; 87-89; 91-93; 95-97; 99-101; 103-105; 107-109; 111-113; 115-117; 119-121; 123-125; 173-175; 177-179; 181-183; 185-187; 189-191; 193-195; 197-199; 67, 68, and 241; 63, 240, and 69; 238, 68, and 242; 239, 68, and 242; and 43, 44, and 243 . Examples of CDRs for the V_{L} chain include SEQ ID NOs: 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 176, 180, 184, 188, 192, 196, and 200. In a further embodiment, the polynucleotide encodes a Fab fragment and a crystallizable fragment (Fc) domain.

In certain embodiments, the polynucleotides described above can be expressed in a supporter cell line. Mammalian cell lines such as Chinese hamster ovary (CHO) cells or 293T cells are particularly suitable for these purposes. The proteins described above are generally soluble, and will therefore be excreted from a producing cell unless they are modified for intracellular retention. Proteins produced in this manner can be purified from the culture medium. Where desired, the proteins may be tagged with (e.g.) a poly-histidine tag or other such commercially common tags to facilitate purification. Proteins produced and purified in this manner can then be administered to a subject in need thereof as described below.

Additionally or alternatively, the polynucleotides described above can be incorporated into a vector (e.g., a transfection vector or a viral transduction vector). Such vectors can then be transfected or transduced into the subject's own cells. In this way, the subject's own cells (e.g., the subject's respiratory mucosa) will produce ACE2 chimeras and/or anti-Spike antibodies to defend these tissues against SARS-CoV-2 infection. Non-limiting examples of vectors comprising the polynucleotides described above are provided herein as SEQ ID NOs: 220-233.

### V. Methods of treatment

The proteins, polynucleotides, and vectors described above can be used to treat and/or prevent COVID-19 disease and/or infection with SARS-CoV-2 or variant thereof. To treat and/or prevent this infection and disease, the proteins, polynucleotides, and vectors described above may be administered to the subject in need thereof in a therapeutically effective amount. The subject may be symptomatic or asymptomatic.

Where a protein is to be administered, any suitable route of administration may be used, including but not limited to intravenous injection, intramuscular injection, subcutaneous injection, and inhalation (e.g. aerosol inhalation). In a particular embodiment, the Fab fragment or polypeptide comprising an ACE2 decoy peptide is administered by inhalation (e.g. aerosol inhalation) to the respiratory mucosa of the subject. In some embodiments, the Fab fragment or polypeptide comprising an ACE2 decoy peptide is supplied to the respiratory mucosa of the subject. Therapeutically effective amounts of these proteins include but are not limited to 1 µg of protein per kg of subject body weight, 5 µg/kg, 10 µg/kg, 50 µg/kg, 100 µg/kg, 500 µg/kg, 1 mg/kg, 5 mg/kg, 10 mg/kg, 50 mg/kg, 100 mg/kg, 500 mg/kg, and 1 mg/kg or more.

Where a polynucleotide is to be transfected, any suitable amount can be administered, including (but not limited to) 10 ng, 50 ng, 100 ng, 500 ng, 1 µg, 5 µg, 10 µg, 50 µg, 100 µg, 500 µg, 1 mg, 5 mg, 10 mg, 50 mg, 100 mg, and 500 mg or more. To transfect subject cells with polynucleotides as described herein, it will be useful to extract cells from the subject, transfect them according to known techniques, and then transfuse the transfected cells back into the subject. Particularly suitable cells circulate throughout the body, such as circulating lymphocytes.

Where a polynucleotide is to be transduced, the viral vector can be administered directly to the subject, or cells can be extracted for transduction and re-transfusion. The viral vector can be administered to the subject by any suitable route of administration, including but not limited to intravenous injection, intramuscular injection, subcutaneous injection, and inhalation (e.g. aerosol inhalation). In a particular embodiment, the viral vector is administered by inhalation (e.g. aerosol inhalation). In another embodiment, the viral vector is supplied to the subject's respiratory mucosa.

Therapeutically effective virus amounts include but are not limited to 1×10⁷ viral particles (VPs), 5×10⁷ VPs, 1×10⁸ VPs, 5×10⁸ VPs, 1×10⁹ VPs, 5×10⁹ VPs, 1×10¹⁰ VPs, or more than 1×10¹⁰ VPs. Adenoviral vectors are particularly suitable for this purpose because of the large cargo capacity of the adenovirus. Suitable adenoviral vectors include those disclosed in WO 98/17783, WO 02/27007, WO 09/6479, & WO 14/31178, each of which is incorporated herein by reference in its entirety. Suitable methods for administering these adenoviral vectors are disclosed in WO 16/112188, which is herein incorporated by reference in its entirety.

Alternatively and/or additionally, administration of a protein or polypeptide (e.g. an ACE2 decoy peptide or a Fab fragment that binds to the SARS-CoV-2 Spike protein) may be combined with a protein or a polynucleotide that encodes a protein that stimulates an immune response to the viral infection (e.g. a SARS-CoV-2 infection). Where appropriate, such treatments include administration of an "IL-15 agonist," (e.g., NAI) an immune stimulatory cytokine or analog thereof (e.g., IL-2), a checkpoint inhibitor, an OX40 ligand, and/or cell lysate to provide a source of DAMPS and PAMPS. The polynucleotides may be a viral vector, such as from a non-pathogenic virus. In some embodiments, the NAI is administered at a dose of about 5 µg/kg/day to about 15 µg/kg/day. In a particular embodiment, the NAI is administered at a dose of about 5 µg/kg/day, about 6 µg/kg/day, about 7 µg/kg/day, about 8 µg/kg/day, about 9 µg/kg/day, about 10 µg/kg/day, about 11 µg/kg/day, about 12 µg/kg/day, about 13 µg/kg/day, about 14 µg/kg/day, or about 15 µg/kg/day. In a specific embodiment, the NAI is administered at about 10 µg/kg/day.

In a still further embodiment, an additional agent that modulates at least one of ACE and ACE2 signaling is administered. The agent that modulates at least one of ACE or ACE2 signaling may be an ACE inhibitor or a compound that increases ACE2 expression. In some embodiments, the compound may be an ACE inhibitor selected from, but not limited to, the group consisting of Benazepril, Captopril, Fosinopril, Lisinopril, Enalapril, Perindopril, Moexipril, Quinapril, Ramipril, and Trandolapril. In some embodiments, the compound is an angiotensin receptor antagonist selected from, but not limited to, Candesartan, Olmesartan, Valsartan, and Losartan. Additionally or alternatively, the additional agent may be an inhibitor of BDKRB1/2, AGTR1, MAS1, or renin.

In another embodiment, treatment of a subject having, diagnosed with having, or suspected of having SARS-CoV-2 comprises administering an IL-15 agonist or IL-2 as the sole therapeutic agent. Alternatively, the IL-15 agonist is administered in combination with another active agent, such as an agent that modulates at least one of ACE or ACE2 signaling. In another embodiment, treatment of a subject infected with, diagnosed as being infected with, or suspected of being infected with COVID-19 comprises an IL-15 agonist or IL-2 as the sole therapeutic agent or in combination with another active agent, such as an agent that modulates at least one of ACE or ACE2 signaling. In a further embodiment, the IL-15 agonist for treating SARS-CoV-2 and/or COVID-19 infection is NAI. In some embodiments, the NAI is administered at a dose of about 5 µg/kg/day to about 15 µg/kg/day. In a particular embodiment, the NAI is administered at a dose of about 5 µg/kg/day, about 6 µg/kg/day, about 7 µg/kg/day, about 8 µg/kg/day, about 9 µg/kg/day, about 10 µg/kg/day, about 11 µg/kg/day, about 12 µg/kg/day, about 13 µg/kg/day, about 14 µg/kg/day, and about 15 µg/kg/day. In a specific embodiment, the NAI is administered at about 10 µg/kg/day.

With respect to the non-pathogenic virus it should be appreciated that all viruses suitable for gene therapy are deemed suitable for use herein. However, especially preferred viruses are those already established in therapy, including adenoviruses, adeno-associated viruses, alphaviruses, herpes viruses, lentiviruses, etc. Among other appropriate choices, adenoviruses are particularly preferred. It should be particularly appreciated that these viruses are suitable for delivery of both payloads, the nucleic acid sequence for the host protein that is critical to viral entry/replication and the nucleic acid sequence for the antigenic viral proteins.

Moreover, it is further generally preferred that the virus is a replication deficient and non-immunogenic virus. For example, suitable viruses include genetically modified alphaviruses, adenoviruses, adeno-associated viruses, herpes viruses, lentiviruses, etc. However, adenoviruses are particularly preferred. For example, genetically modified replication defective adenoviruses are preferred that are suitable not only for multiple vaccinations but also vaccinations in individuals with preexisting immunity to the adenovirus (see e.g., WO 2009/006479 and WO 2014/031178, which are incorporated by reference in its entirety). In some embodiments, the replication defective adenovirus vector comprises a replication defective adenovirus 5 vector. In some embodiments, the replication defective adenovirus vector comprises a deletion in the E2b region. In some embodiments, the replication defective adenovirus vector further comprises a deletion in the E1 and/or E3 region. In that regard, it should be noted that deletion of the E2b gene and other late proteins in the genetically modified replication defective adenovirus to reduce immunogenicity. Moreover, due to these specific deletions, such genetically modified viruses were replication deficient and allowed for relatively large recombinant cargo.

### EXAMPLES

The following example is provided to further illustrate the invention disclosed herein but, of course, should not be construed as in any way limiting its scope.

**Example 1**: Selected exemplary constructs presented herein were expressed in CHO-S using Maxcyte electroporation. 3.2×10⁸ cells were electroporated in Maxcyte electroporation buffer and cultured in CD Opti CHO media with CD CHO efficient feed for 14 days at 32°C and 3% CO₂. ACE2-Fc hybrid constructs were isolated following known isolation procedures. The ACE2-Fc IgG Hybrid Protein had an amino acid sequence of SEQ ID NO:234, while the ACE2-Fc IgG R273Q Hybrid Protein (lacking ACE2 activity) had an amino acid sequence SEQ ID NO:235. The ACE2-Fc IgA Hybrid Protein had an amino acid sequence of SEQ ID NO:236, while the ACE2-Fc IgA R273Q Hybrid Protein (lacking ACE2 activity) had an amino acid sequence SEQ ID NO:237.

Yield and relative purity for a small-scale Maxcyte production of IgG-Fc hybrid constructs were in a desirable range. Table 4 provides numerical results for the size exclusion chromatography.

| Table 4 | | | |
|---|---|---|---|
| **ID** | **Production Culture Volume (mL)** | **Yield** | **% Main on SEC-HPLC** |
| ACE2-IgGlFc | 30 | 5.2 | 90.9 |
| ACE2(R273Q)-IgG1Fc | 30 | 8.4 | 91.7 |
| ACE2-IgG1Fc-Avi-tag | 40 | 6.2 | 94.5 |

ACE2-IgAFc expression was as efficient as ACE2-IgG₁Fc expression, with no apparent differences in the mutated form (R273Q) vs. non-mutated form. Traditional IgG purification processes are often not effective for IgA, so the hybrid constructs were isolated using ion exchange chromatographic and affinity media selective for IgA. The purified ACE2-Fc hybrid constructs were tested for binding and avidity against 2019-n-CoV Spike protein. Table 5 lists exemplary test results.

| Table 5 | | | | | |
|---|---|---|---|---|---|
| **Method** | **Ligand** | **Analyte** | **kₒₙ (1/Ms)** | **k_{off} (1/s)** | **KD (nM)** |
| Octet-SA sensor | Spike-RBD | ACE2-IgG₁Fc | 1.03E+05 | 7.89E-05 | 0.76 |
| Octet-SA sensor | Spike-RBD | ACE2(R273Q)-IgG₁Fc | 1.04E+05 | 9.86E-05 | 0.95 |
| Octet-SA sensor | Spike-RBD | rACE2(1-740aa) dimer | 5.37E+04 | 6.63E-05 | 1.2 |
| SPR | 2019-nCoV Spike | ACE2 (1-615aa) monomer | 1.88E+05^{∗} | 2.76E-03^{∗} | 14.7^{∗} |

Where modified ACE2-Fc hybrid constructs are used for diagnostic tests, multiple test formats can be chosen. One exemplary system uses a biotin tagged ACE2-Fc hybrid construct immobilized to MSD streptavidin plate and another modified ACE2-Fc hybrid construct for detection (sulfo-tag MSD label). As shown, the ACE2-Fc hybrid constructs have high sensitivity and specificity.

**Example 2:** To confirm the affinities of various anti-Spike antibodies disclosed herein for the SARS-CoV-2 Spike, KD was determined by surface plasmon resonance (SPR). All SPR experiments were carried out on Pioneer FE (Sartorius Corporation) and the data were analyzed by Qdat analytical software. Antibodies were captured on the SPR sensor coated with anti-Human IgG Fc. OneStep kinetic assay was performed using 50 nM RBD-SD1 or 20 nM sc-S-PP-T4F samples. The mAbs whose ability to bind Spike trimer and/or RBD peptide are indicated in Tables 1 & 2 above.

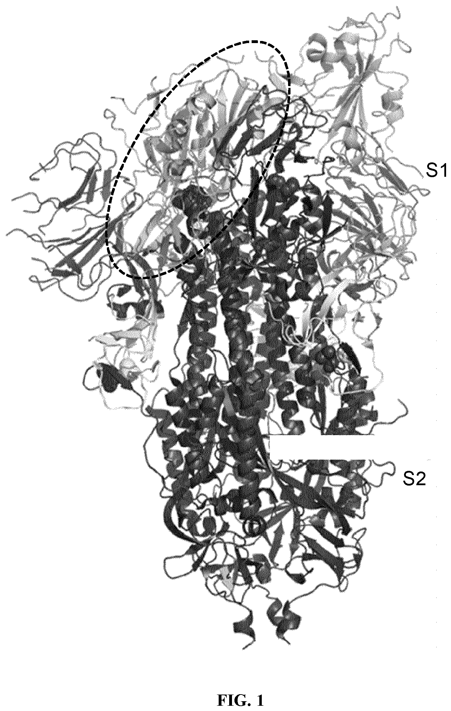

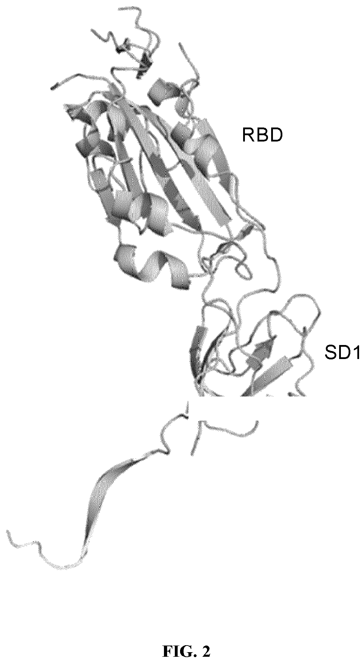

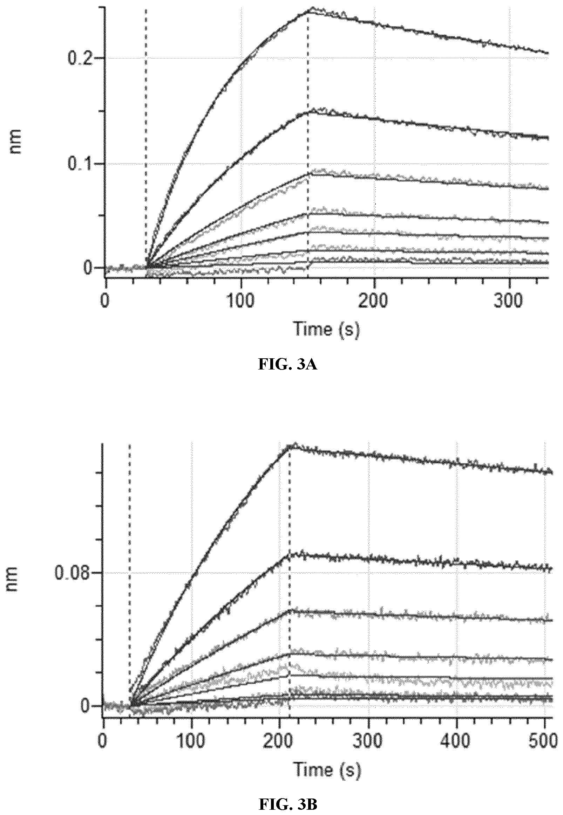

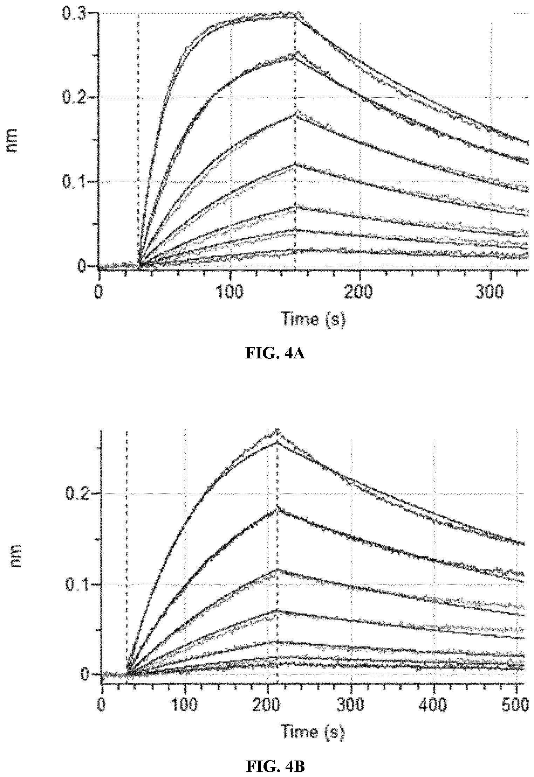

**Example 3:** To study epitope of antibodies, sc-S-PP-T4F, RBD-SD1, and RBD were used as analytes. These various constructs are illustrated in FIGs. 1 & 2. All epitope analyses were run on Octet Red96e (Sartorius Corp.). Antibodies were captured on anti-Human Capture (AHC) biosensors. A 2× dilution series starting at 20 nM sc-S-PP-T4F and another starting at 100 nM RBS-SD1 and RBD were used for kinetic assay cycle. Table 3 below summarizes these epitope determination trials with a select few mAbs. FIGs. 3 & 4 show SPR plots from some of these trials. N-612-004 and N-612-017 each bind cleaved Spike peptide with 1:1 binding mode, which was observed with binding RDB-SD1, indicating that cleaved Spike contains only S1 domain. The calculated k_{off} for the N-612-004 against cleaved Spike is 9.8×10⁻⁴, for N-612-004 against RBD-S1 is 3.4×10⁻⁴, for N-612-017 against cleaved Spike is 3.9×10⁻³, and for N-612-017 against RBD-S1 is 1.9×10⁻³.

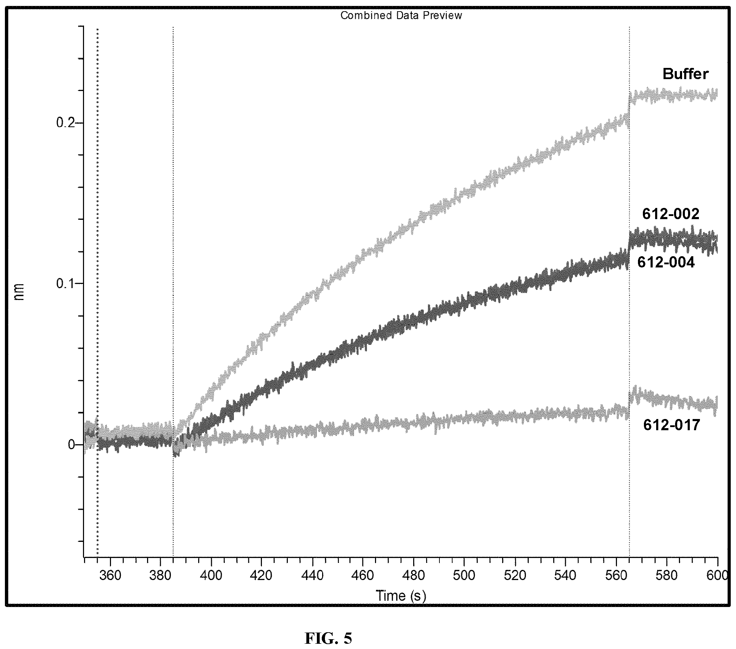

**Example 4:** Ni-NTA biosensors were coated with His-tagged RBD-SD1 and quenched with anti-RBD-SD1 antibodies. Immediately after quenching, the sensors were incubated with ACE2-IgG1Fc solution to determine which antibody blocked ACE2-IgGFc binding to RBD-SD1 on sensors. In FIG. 5, N-612-004 binds both S and S-RBD-SD1, while N-612-002 only binds S. In other words, N-612-002 binds RBD or Spike but does not interfere with ACE2 binding. Steric hindrance likely explains the low N-612-002 binding. The overlapping N-612-002 and N-612-004 lines run discernibly underneath the line for buffer, showing *some* obstruction of ACE2/RBD binding. Notably, mAb N-612-017 is able to obstruct Spike binding to ACE2 almost completely in these *in vitro* conditions.

| Table 6. Anti-Spike IgG1 binding analysis summary (ND = not determined) | | | | | |
|---|---|---|---|---|---|
| **IgG** | **Sc-S-PP-T4F (uncleaved S)** | **S-T4F (cleaved S)** | **RBD-S1** | **RBD** | **ACE2 blocking** |
| N-612-002 | Yes (Avidity) | No | No | No | No |
| N-612-004 | Yes (Avidity) | Yes (1:1 binding) | Yes (1:1 binding) | No | No |
| N-612-007 | Yes (Avidity) | Yes (Avidity) | No | No | No |
| N-612-014 | Yes (Avidity) | Yes (1:1 binding) | No | No | No |
| N-612-017 | Yes (Avidity) | Yes (1:1 binding) | Yes (1:1 binding) | Yes (1:1 binding) | Yes |
| N-612-041 | Yes (Avidity) | ND | Yes (1:1 binding) | No | No |
| N-612-044 | Yes (Avidity) | ND | No | No | No |
| N-612-074 | Yes (Avidity) | ND | Yes (1:1 binding) | Yes (1:1 binding) | ND |

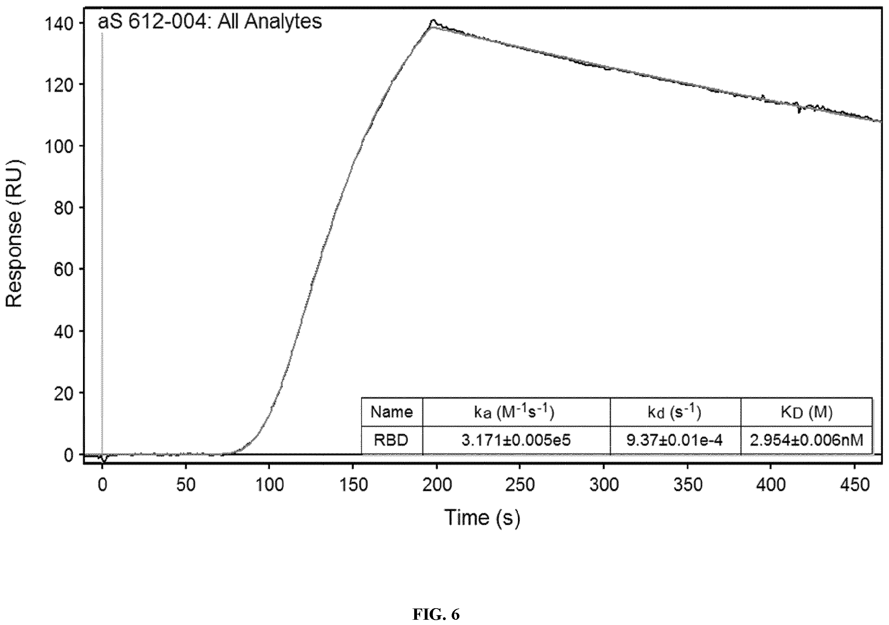

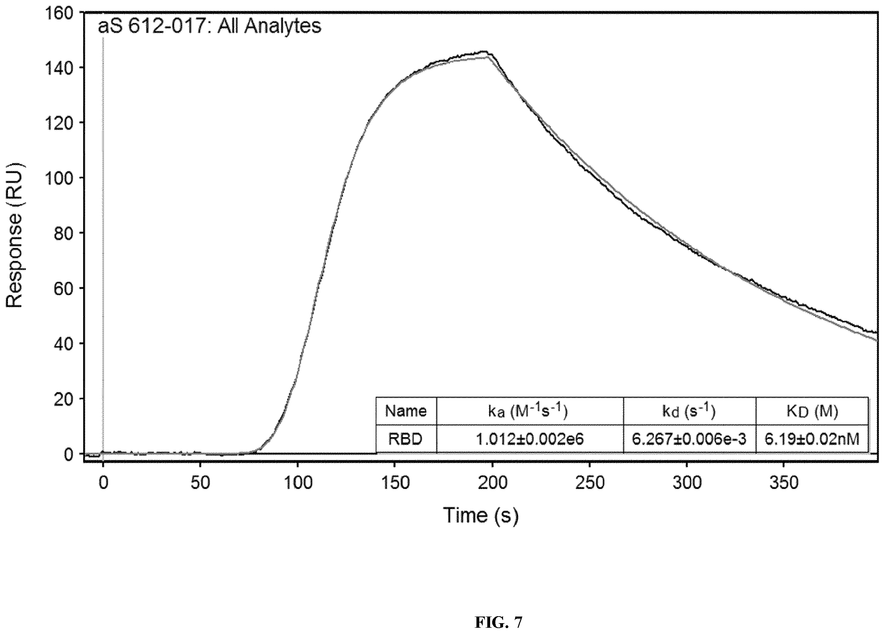

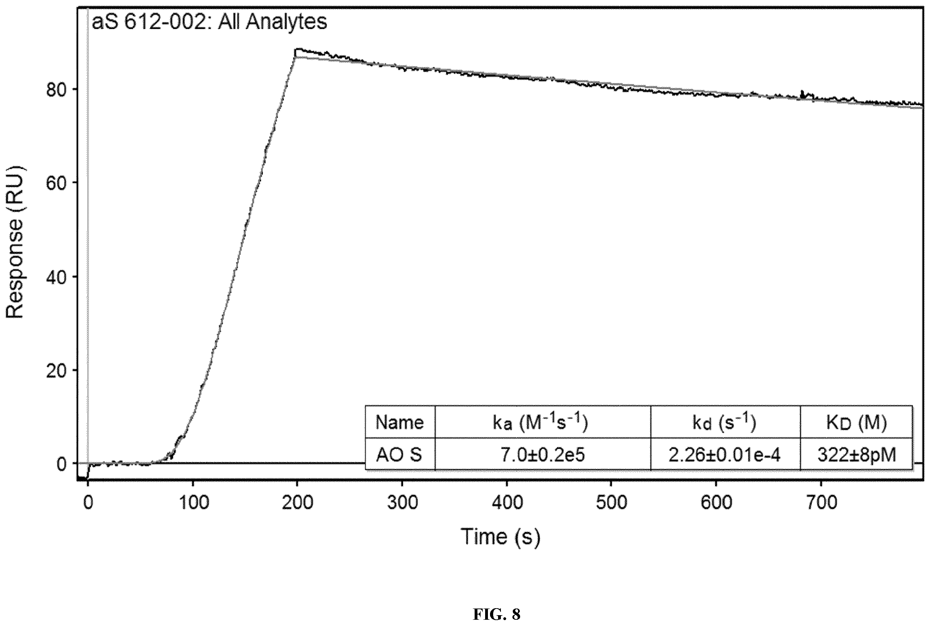

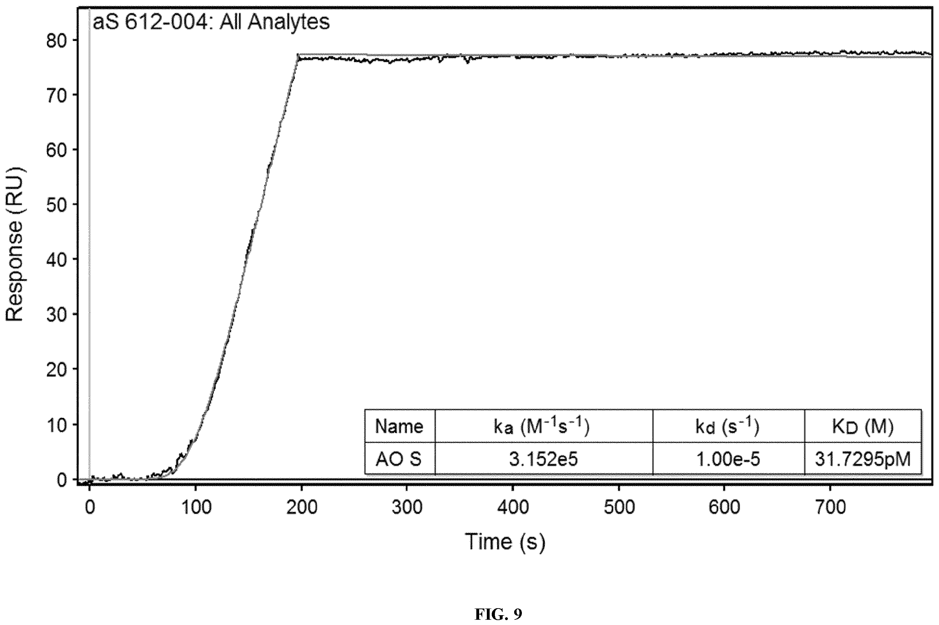

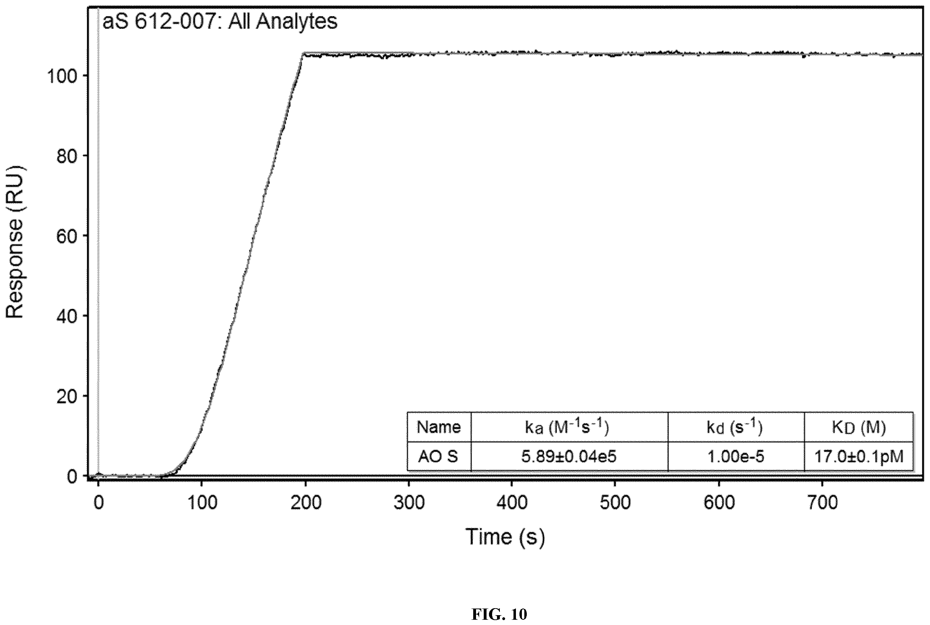

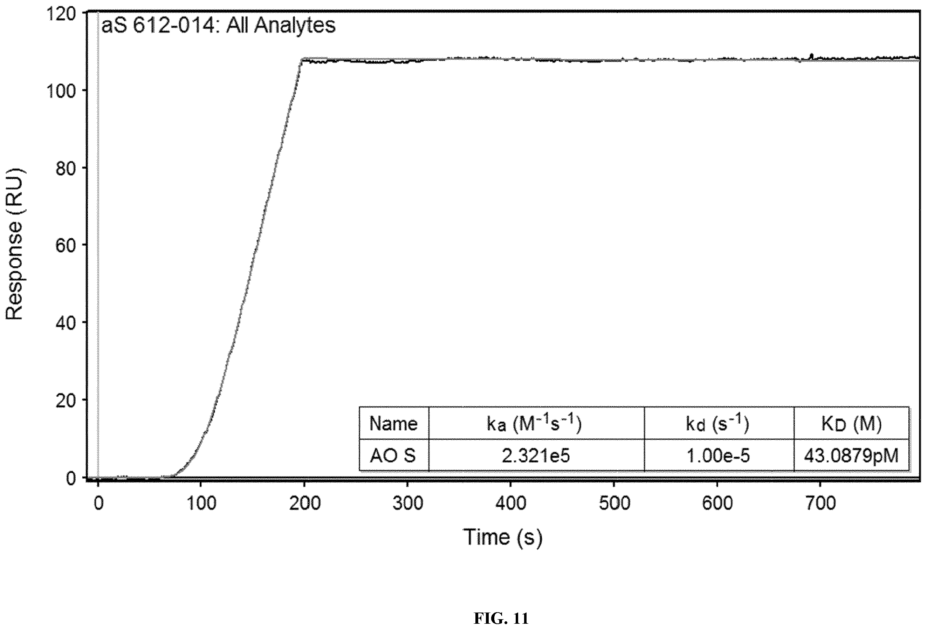

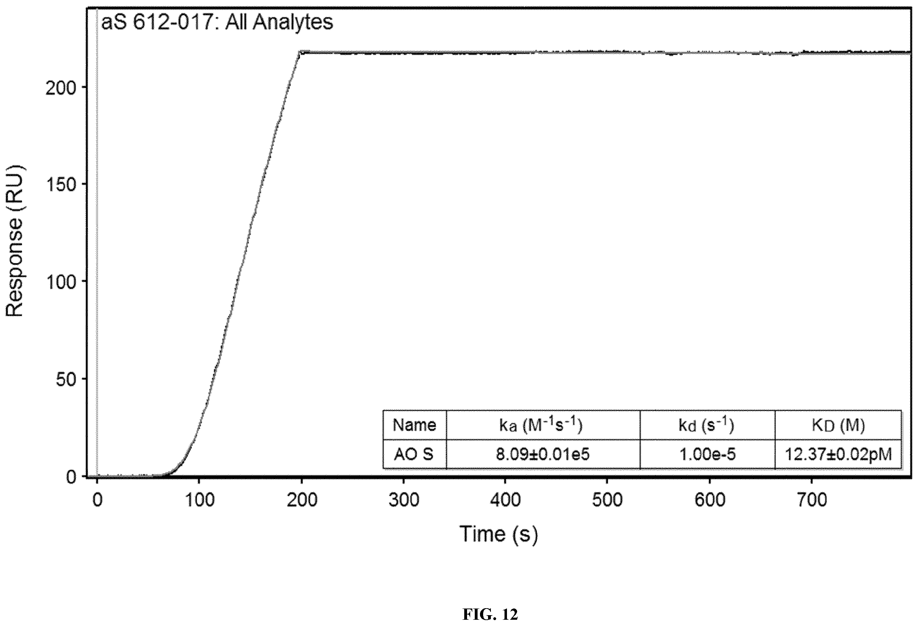

**Example 5:** A Pioneer FE instrument was used to measure the binding affinities between IgG1 and the recombinant spike trimer ectodomain or RBD-SD1 protein. SPR affinity curves for two anti-spike IgG1 s against the RBD-SD 1 peptide show K_{D} determinations in FIGs. 6 and 7. SPR affinity curves for a selection of anti-spike IgG1s against Sc-S-PP-T4F show K_{D} determinations in FIGs. 8-12. Spike binding data is avidity enhanced resulting in flat dissociation curves.

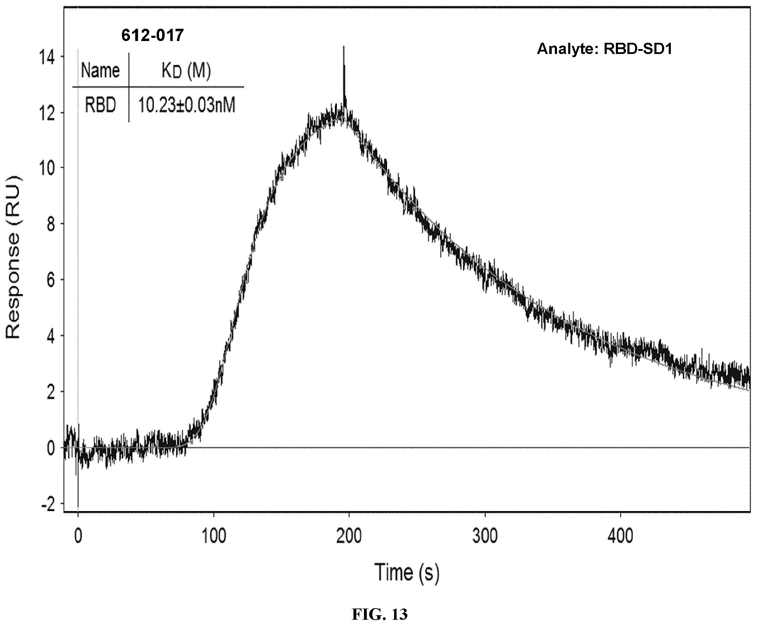

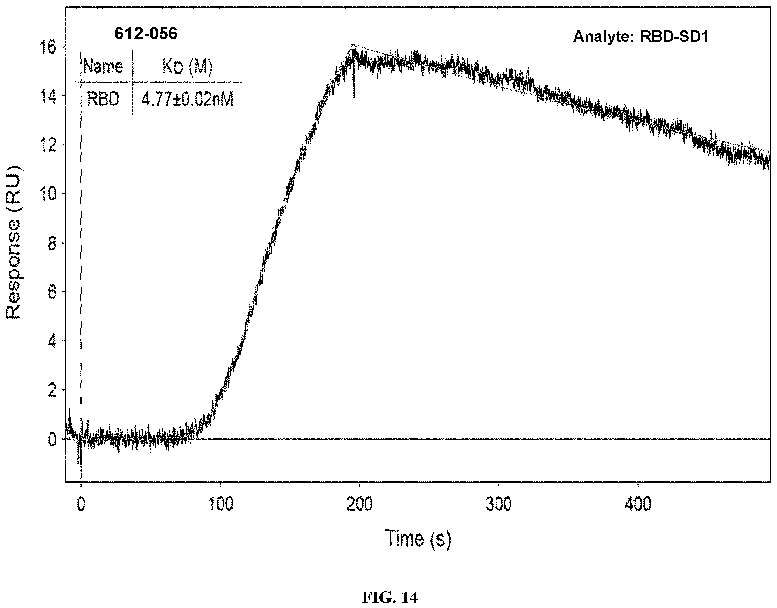

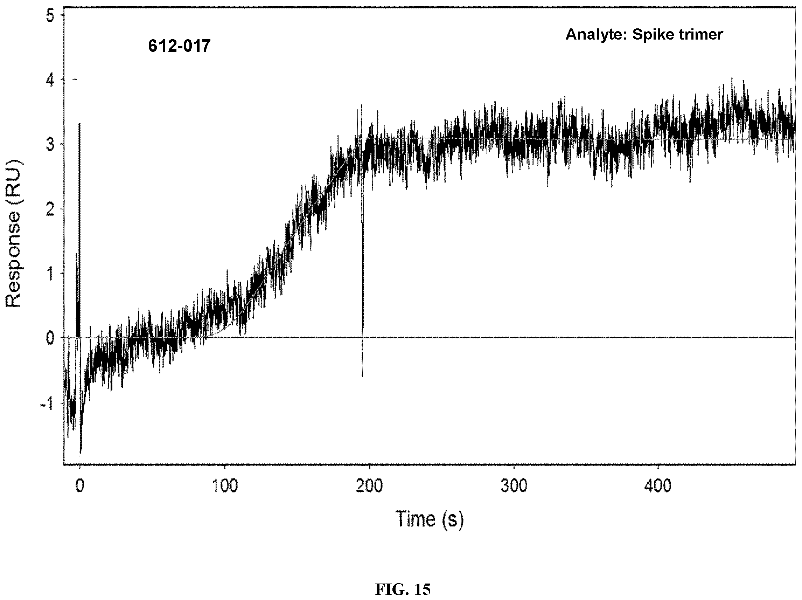

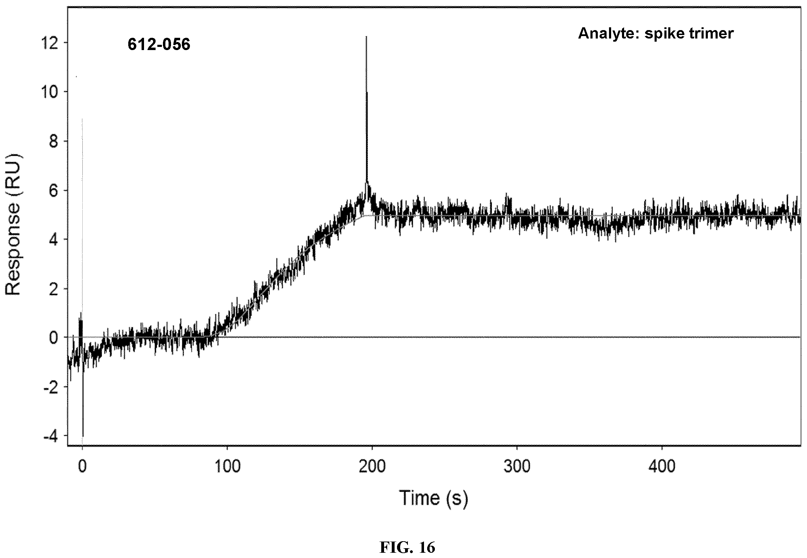

**Example 6:** Binding affinities between scFv and the recombinant spike trimer ectodomain or RBD-SD1 protein were measured on a Pioneer FE instrument. Briefly, biotinylated anti-FLAG M2 antibody was immobilized onto the PCH biosensor (Molecular Devices/ForteBio) coated with neutravidin. Triple-FLAG-tagged scFvs were captured with anti-FLAG M2 on the sensor and the binding affinities were measured using OneStep injection with the recombinant analyte protein. Avidity effects with the spike trimer result in a flat dissociation curve that was not fitted. SPR affinity curves for two anti-spike scFvs against the RBD-SD1 peptide show K_{D} determinations in FIGs. 13 and 14. SPR affinity curves for the anti-spike scFvs against SARS-CoV-2 Spike trimers show K_{D} determinations in FIGs. 15 and 16.

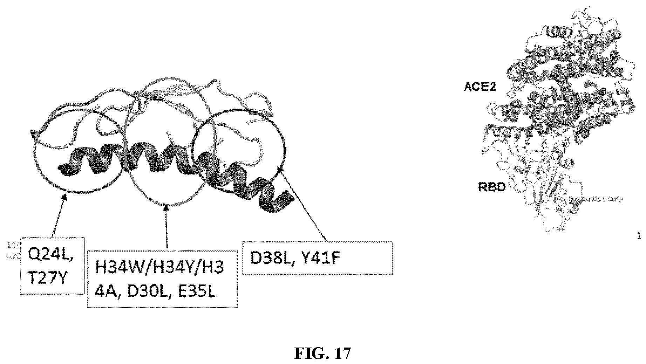

**Example 7**: RBD structure can be likened to a human foot, with "heel," "arch," and "toe" regions. Close analysis of the binding interface reveals a pocket between the "arch" portion and the ACE2 α-helix. Water could be displaced from this pocket to increase binding strength between RBD and the ACE2 α-helix. Mutants of this α-helix could be used as competitive inhibitors of ACE2/RBD binding, or as a molecular trap to sequester RBD from cell-surface ACE2. FIG. 17 and Table 7 illustrate an ACE2-IgG₁Fc mutation study confirming that the 'heel' is the most important element for binding affinity to RBD. While the H34A mutant ACE2 α-helix was shown to bind to the RBD with a Kd of 9.4 nM, and the T27Y mutant ACE2 α-helix was shown to bind to the RBD with a Kd of 3.8 nM, the presence of a double mutation, H34A and T27Y creates a synergistic effect and increases the binding affinity to about 1 nM.

| **Table 7** | | | | | | |
|---|---|---|---|---|---|---|
| **Mutants** | **Plasmid** | **Target basis** | **Anticipated effect of mutation** | **kon (1/Ms)** | **koff (1/s)** | **KD (nM)** |
| WT | | | | 3.10E+05 | 6.20E-03 | 19.6 |
| Q24L | SR1 | Q H-bonds with A and N on Spike | Predicted to not change binding kinetics | 5.70E+05 | 1.90E-02 | 32.6 |
| T27Y | SR3 | | Predicted to not change binding kinetics | 4.00E+05 | 3.70E-03 | 9.4 |
| H34W | SR5 | H34 important per MD but not EM | Predicted to further space fill & increase binding kinetics | 5.60E+05 | 2.20E-02 | 38.6 |
| H34Y | SR7 | H34 important per MD but not EM | Predicted to further space fill & increase binding kinetics | 4.60E+05 | 3.90E-02 | 84.7 |
| H34A | SR9 | H34 important per MD but not EM | Predicted to reduce binding kinetics | 2.30E+05 | 8.40E-04 | 3.6 |
| D30L | SR11 | 50% H-bond to K in Spike in MD, none in EM | Predicted to reduce binding kinetics | 2.80E+05 | 9.90E-03 | 34.9 |
| E35L | SR13 | E H-bonds | Predicted to reduce binding kinetics | 4.80E+05 | 1.10E-02 | 22.8 |
| H34InsAE35 | SR15 | | Predicted to further space fill & increase binding kinetics; distinguish importance of space fill vs H-bond as this insertion may impact H34 & E35 H-bonds | -- | -- | Very weak |
| D355L | SR17 | 64% H-bond to T in Spike | Predicted to reduce binding kinetics | 5.20E+05 | 4.10E-02 | 80 |
| D38L | SR19 | 36% interaction with Y on Spike | Predicted to reduce binding kinetics | -- | -- | very weak |
| Y41F | SR21 | 19% H-bond | Predicted to reduce binding kinetics | -- | -- | No binding |

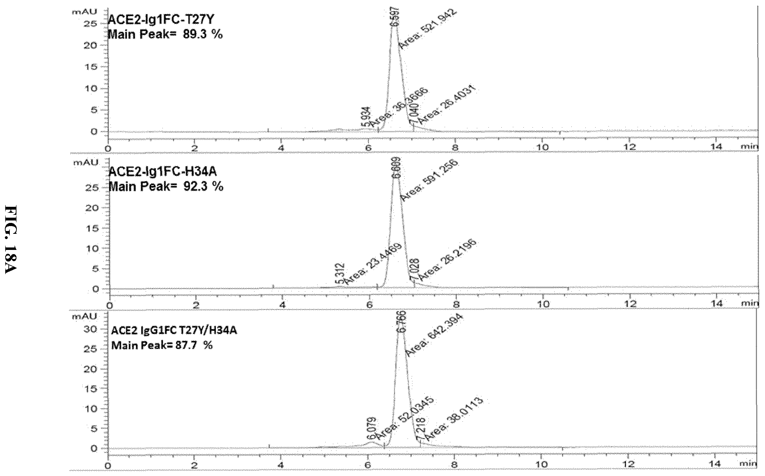

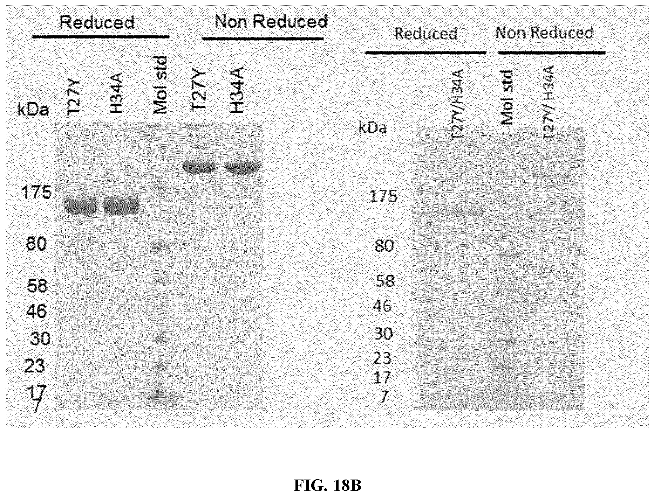

FIGs. 18A and 18B illustrate that ACE2-IgG1Fc mutants were successfully expressed and purified. The purification summary in Table 8 shows the purification of single point mutations H34A and T27Y as well as the double mutant (H34A and T27Y).

| **Table 8** | | | |
|---|---|---|---|
| **Sample** | **Final Titer (µg/mL**) | **Conc. (mg/mL)** | **Mass (mg)** |
| T27Y | 39.0 | 0.94 | 0.9 |
| H34A | 39.1 | 1.1 | 0.9 |
| T27Y/H34A | 15.6 | 1.1 | 1.1 |

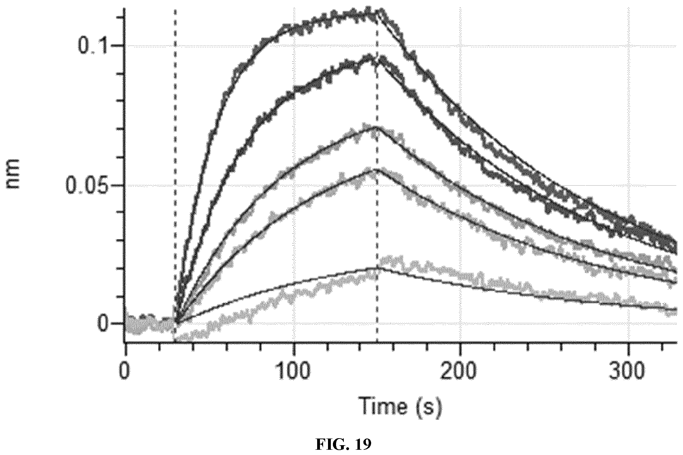

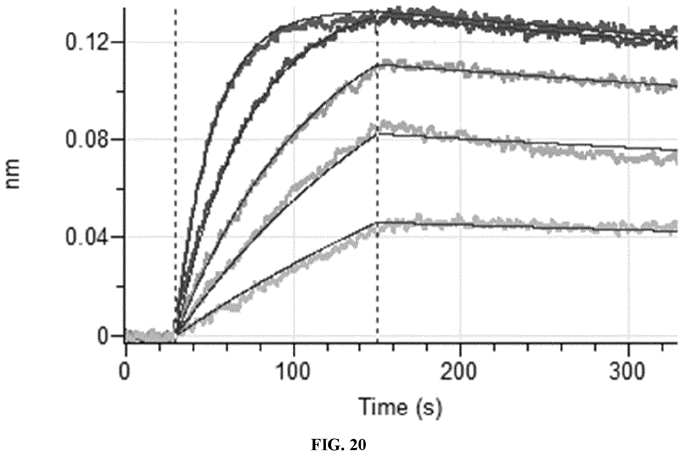

**Example 8:** The binding affinities of wildtype ACE2-IgG₁Fc antibody, the T27Y and H34A single mutants, and the T27Y/H34A double mutant ACE2-IgG₁Fc antibody were measured. The antibodies were captured on AHC biosensors as in Example 3. A two-fold dilution series starting at 200 µM RBD-SP1 was used for kinetic assay cycle. FIGs. 19 and 20 show SPR plots from some of these trials. Table 9 below summarizes binding data for the two antibodies.

| **Table 9**: Binding data for wildtype and ACE2 mutant antibodies | | | | |
|---|---|---|---|---|
| **Antibody** | **kₒₙ (1/Ms)** | **k_{off} (1/s)** | **k_{D} (nM)** | **Virus neutralization IC₅₀ (µg/mL)** |
| ACE2(WT)-IgG₁Fc | 3.5×10⁵ | 7.4×10⁻³ | 21.4 | 3.2 |
| ACE2(T27Y)-IgG₁Fc | 2.9×10⁵ | 1.2×10⁻³ | 4.1 | ND |
| ACE2(H34A)-IgG₁Fc | 5.1×10⁵ | 4.1×10⁻³ | 8.0 | ND |
| ACE2(T27Y/H34A)-IgG₁Fc | 4.6×10⁵ | 4.6×10⁻⁴ | 1.0 | 0.24 |

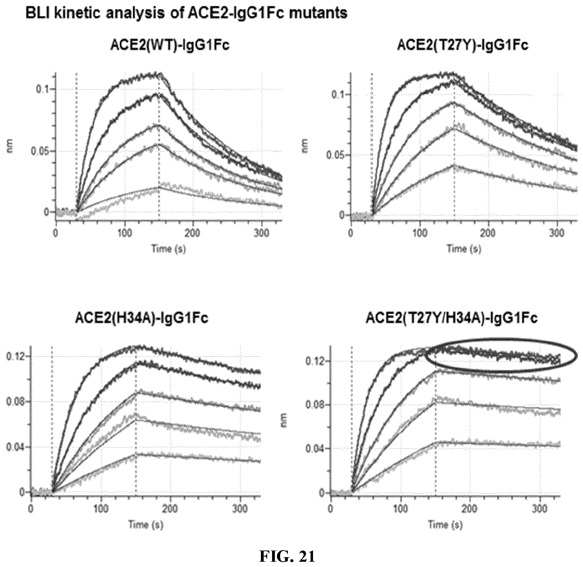

FIG. 21 illustrates that ACE2-IgG₁Fc double mutations (T27Y/H34A) has superior binding affinity for RBD as compared to wildtype ACE2-IgG₁Fc and ACE2-IgG₁Fc single mutants.

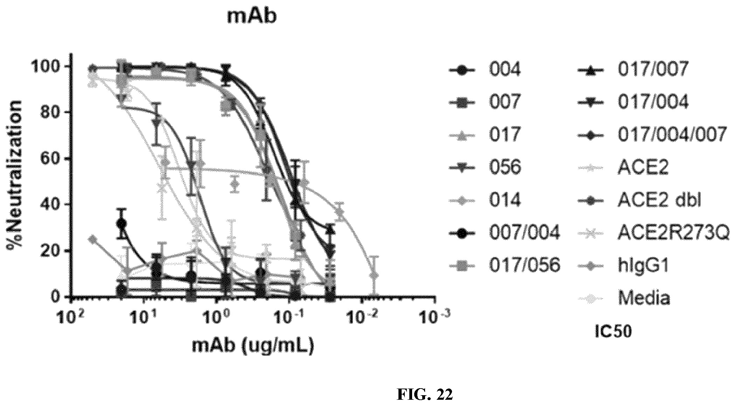

**Example 9**: FIG. 22 illustrates live SARS-CoV-2 neutralization data. The neutralization of SARS-CoV-2 was tested against wild type and mutant ACE2-IgG₁Fc. Wild type ACE2-IgG₁Fc as well as its enzyme inactivation mutation (R273Q) were fairly adequate in neutralizing SARS-CoV-2. ACE2-IgG₁Fc with H34A and T27Y mutations (ACE2 dbl) was much better in neutralizing SARS-CoV-2 than the wild type variant. The ACE2 double mutant, having the H34A and T27Y mutations, was as good as antibody 017 in neutralizing SARS-CoV-2. Table 10 reports the IC₅₀ values of various neutralizing antibodies and ACE2 decoy peptides calculated from the neutralization experiment.

| **Table 10** | | | | |
|---|---|---|---|---|
| **Antibody /Decoy** | **IC₅₀ (µg/mL)** | | **Antibody /Decoy** | **IC₅₀ (µg/mL)** |
| N-612-004 | 0.1940 | | N-612-017/ N-612-004 | 0.094 |
| N-612-007 | 0.04515 | | N-612-017/ N-612-004/ N-612-007 | 0.04563 |
| N-612-017 | 0.07556 | | ACE2 decoy | 1.616 |
| N-612-056 | 0.8549 | | ACE2 dbl decoy | 0.1076 |
| N-612-014 | 0.007907 | | ACE2 R273Q decoy | 3.029 |
| N-612-017/ N-612-056 | 0.06423 | | hIgG₁ | no activity |
| N-612-017/ N-612-007 | 0.04519 | | Media | no activity |

The combination of ACE2-IgG₁Fc or ACE2 IgA with H34A and T27Y expressed as an adenovirus vaccine with various antibodies was also tested for neutralization ability. It was found that combining antibodies N-612-017 with either N-612-007 (binds S2) or N-612-004 (binds SD1) or both together improves neutralization though neither N-612-004 nor N-612-007 had much neutralization activity. Finally, combining N-612-017 with N-612-014 and/or N-612-056 was found to significantly improve neutralization.

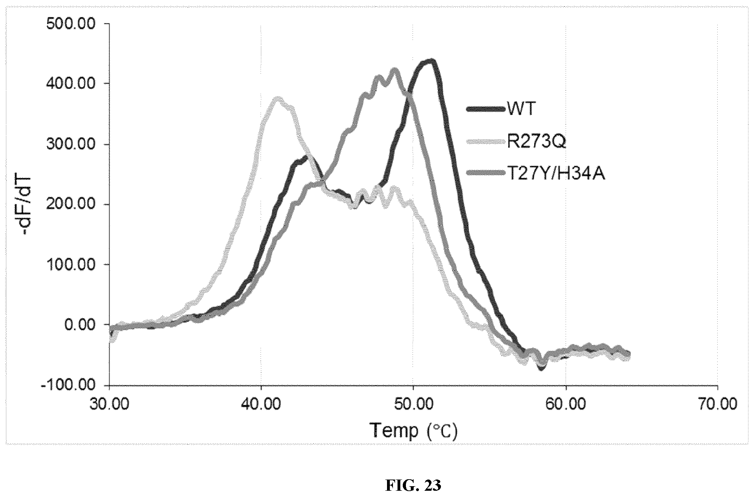

**Example 10:** The stabilities of wildtype, R273Q mutant, and T27Y/H34A double mutant ACE2 decoy peptides were measured by differential scanning fluorimetry (DSF). 20 µL of 1 mg/mL antibody was mixed with 10 µL of 20X SYPRO orange. The plate was scanned from 20°C to 70°C at 0.5°C increment in a CFX96 Real-Time System (BioRad). The DSF results for the wildtype, R273Q mutant, and T27Y/H34A double mutant ACE2 decoy peptides are shown in FIG. 23.

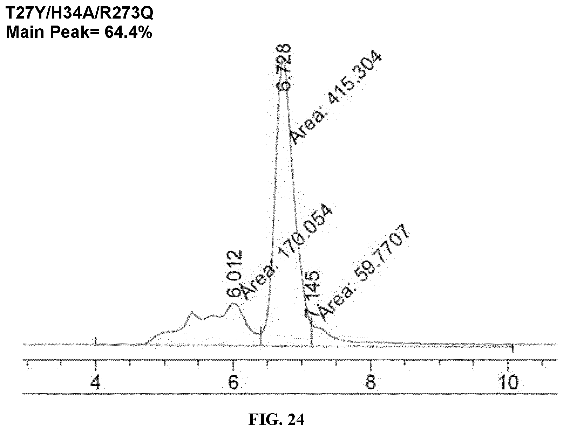

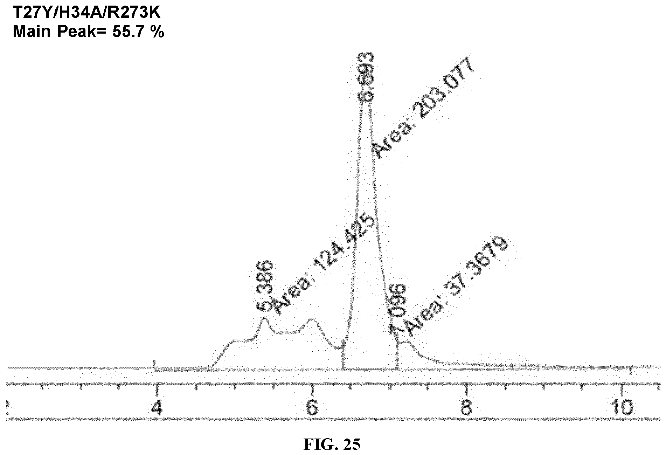

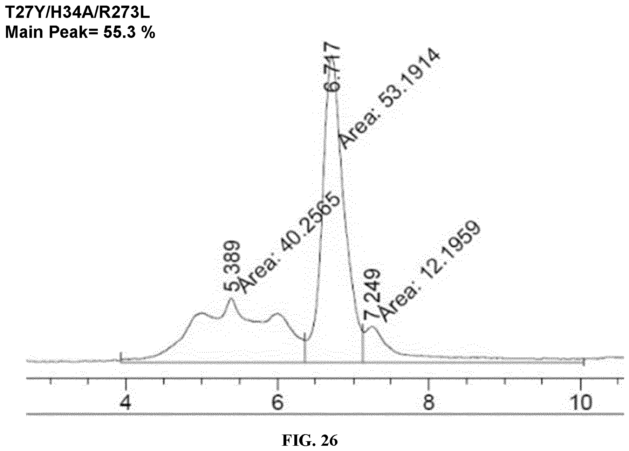

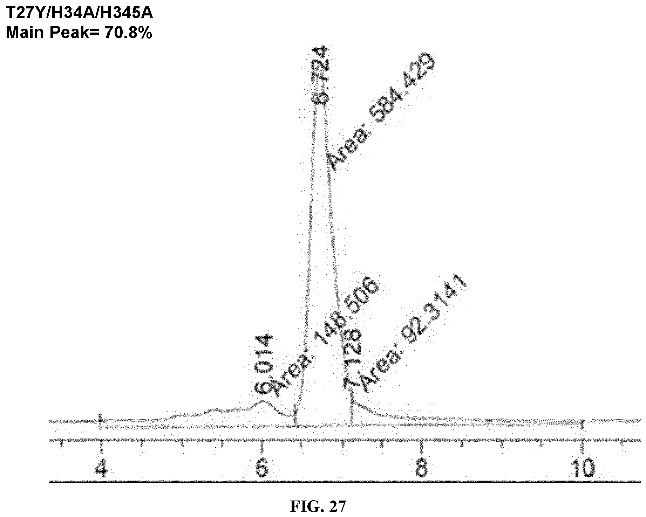

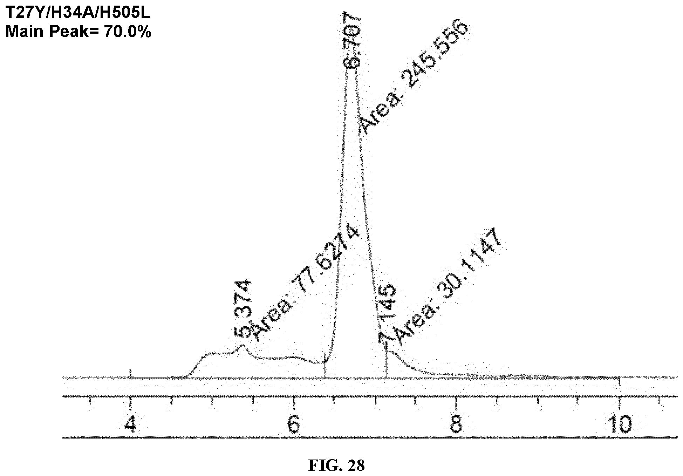

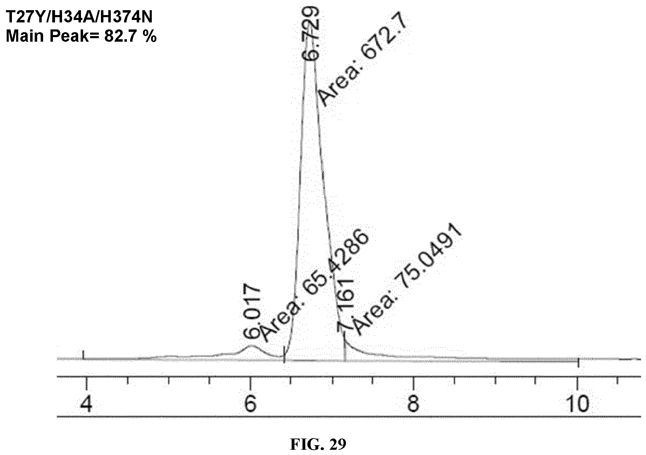

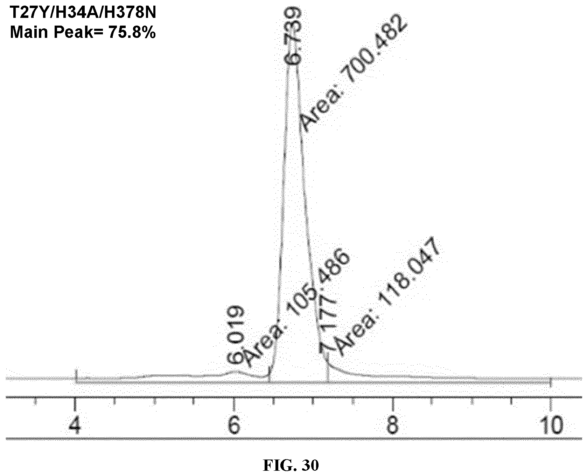

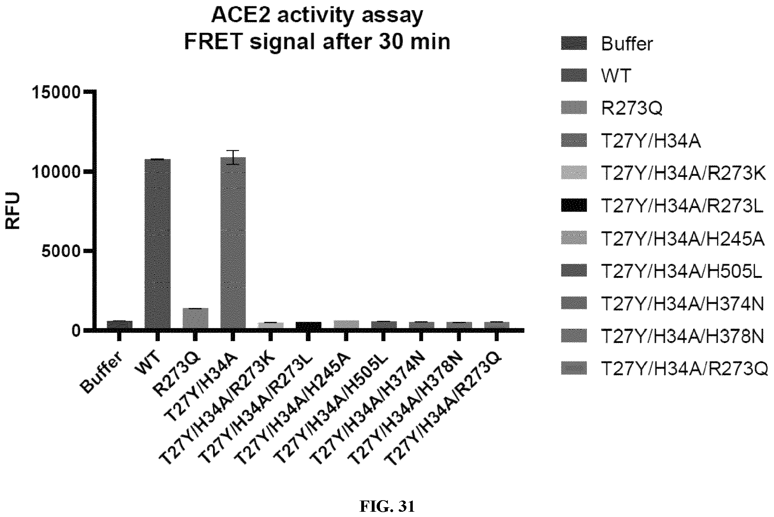

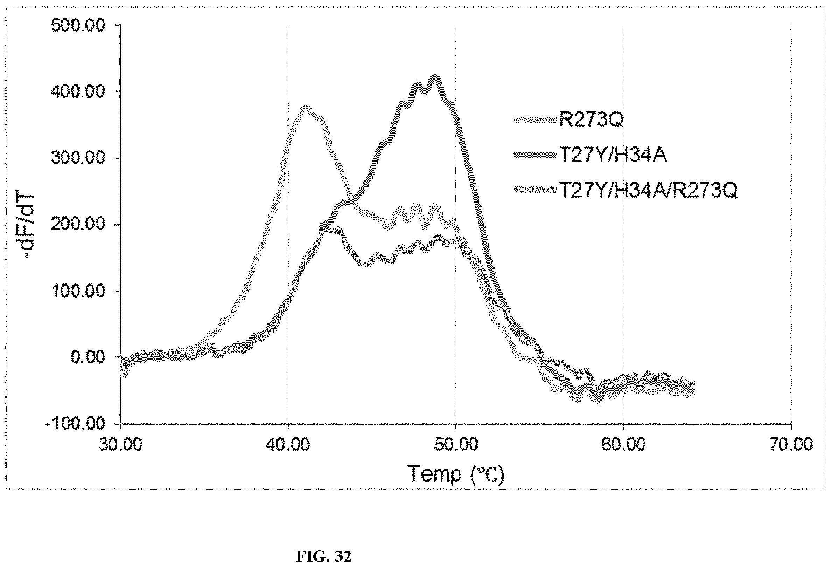

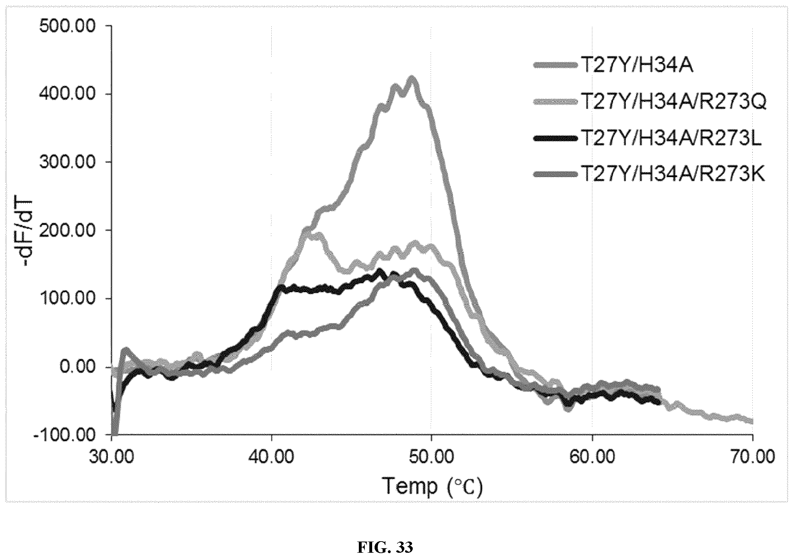

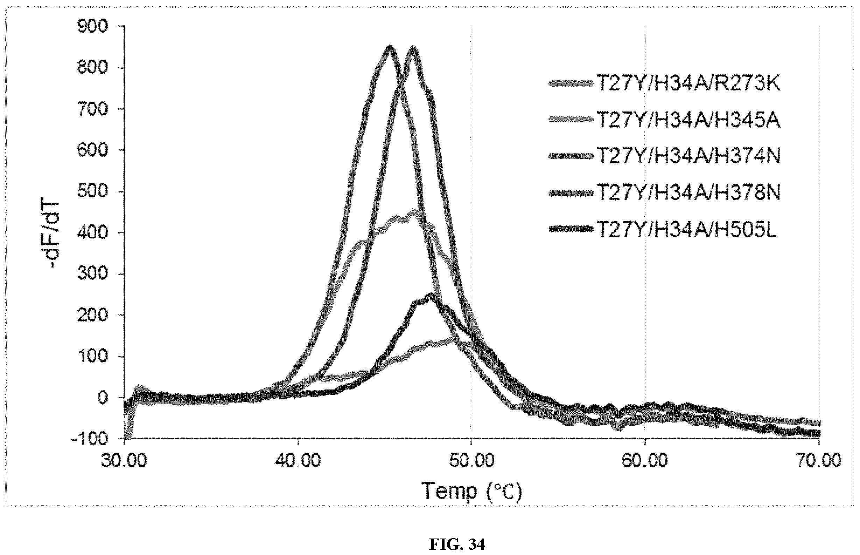

**Example 11:** Seven triple mutant ACE2 decoy peptides were prepared. The size of each triple mutant ACE2 decoy peptides was measured by size exclusion chromatography. Briefly, purified triple mutant ACE2 decoy peptides were run on a Zenix-C SEC-300 (Sepax), 3 µm, 7.8 × 300 mm in a running buffer containing 50 mM sodium phosphate, 250 mM sodium chloride, and a pH of 6.8. FIGs. 24-30 show the sizes of each triple mutant ACE2 decoy peptide. The ACE2 activities of wildtype ACE2 decoy peptide, R273Q mutant ACE2 decoy peptide, T27Y/H34A double mutant ACE2 decoy peptide and each triple mutant ACE2 decoy peptide were also determined. 1 µg/mL of each sample was mixed with 20 µM substrate (MCA-Tyr-Val-Ala-Asp-Pro-Lys(DNP)-OH (R&D Systems) in 75 mM HEPES, 1 M sodium chloride and pH 7.4. The samples were incubated with shaking (700 rpm) for 30 minutes at room temperature. The FRET signal was measured (top read) at excitation and emission wavelengths of 320 nm and 405 nm, respectively. FIG. 31 shows the ACE2 activity of the wildtype and mutant ACE2 decoy peptides. Table 11 summarizes the binding data for the triple mutant ACE2 decoy peptides. The stabilities of various triple mutant ACE2 decoy peptides was measured and compared to single and double ACE2 mutant decoy peptides. DSF was performed as described in Example 7 above. FIG. 32 shows the stabilities of the R273Q, T27Y/H34A, and T27Y/H34A/R273Q mutant ACE2 decoy peptides. FIG. 33 shows the stabilities of three triple mutant ACE2 decoy peptides as compared to the double mutant ACE2 decoy peptide. FIG. 34 shows the stabilities of five triple mutant ACE2 decoy peptides.

| Table 11: Binding data for T27Y/H34A/XXX triple mutant ACE2 decoy peptides | | | |
|---|---|---|---|
| **Antibody** | **kₒₙ (1/Ms)** | **k_{off} (1/s)** | **k_{D} (nM)** |
| T27Y/H34A | 3.77 × 10⁵ | 2.11 × 10⁻⁴ | 0.56 |
| T27Y/H34A/R273K (206) | 4.04 × 10⁵ | 1.79 × 10⁻⁴ | 0.44 |
| T27Y/H34A/R273L (207) | 4.06 × 10⁵ | 4.04 × 10⁻⁴ | 1.00 |
| T27Y/H34A/H345A (211) | 4.15 × 10⁵ | 3.95 × 10⁻⁴ | 0.95 |
| T27Y/H34A/H505L (208) | 4.09 × 10⁵ | 2.91 × 10⁻⁴ | 0.71 |
| T27Y/H34A/H374N (209) | 3.88 × 10⁵ | 2.44 × 10⁻⁴ | 0.63 |
| T27Y/H34A/H378N (210) | 3.95 × 10⁵ | 3.32 × 10⁻⁴ | 0.84 |
| T27Y/H34A/R273Q (212) | 4.86 × 10⁵ | 4.09 × 10⁻⁴ | 0.84 |

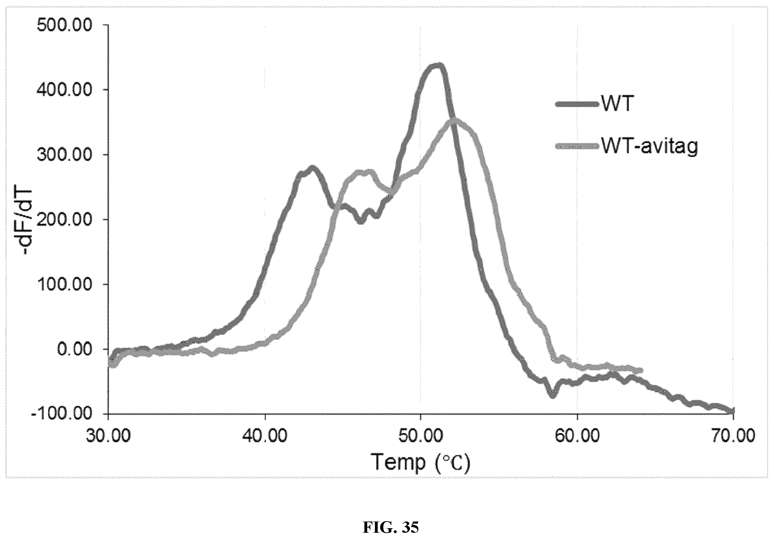

**Example 12:** Wildtype ACE2 decoy peptide was fused to avidin at the C-terminal of the antibody. The stability of the avidin-tagged and untagged wildtype ACE2 decoy peptide was measured by DSF as described in Example 7. FIG. 35 shows the stability of the avidin-tagged and untagged wildtype ACE2 decoy peptide.

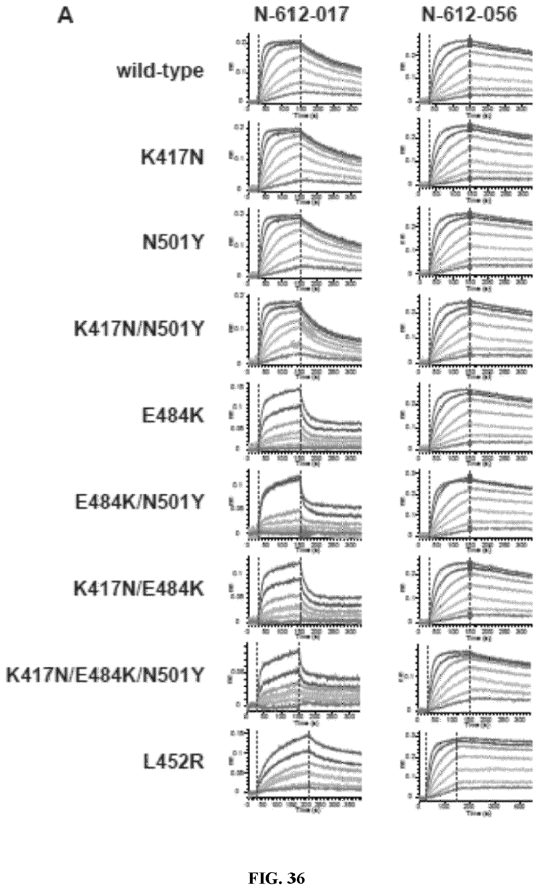

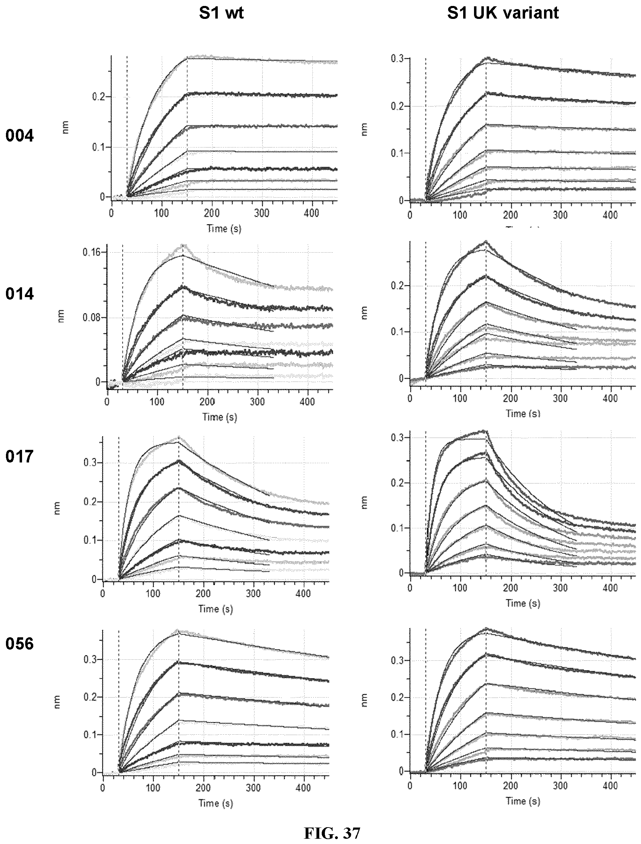

**Example 13**: Anti-Spike antibodies were exposed to wildtype and mutant Spike proteins and the binding affinities of the antibodies for the Spike proteins were measured. All experiments used a running buffer composed of 10 mM HEPES, pH 7.4, 150mM NaCl, 0.02% tween 20, 0.1% BSA unless indicated otherwise. For 1:1 binding affinity determination of mAbs against SARS-CoV-2 RBD wild-type and mutants and S1 variant, mAbs were immobilized onto AHC sensor (Sartorius Corporation), and the RBD (wild-type or mutants) and S1 concentration series of 200, 100, 50, 25, 12.5, 6.25, 3.125 nM were tested to determine K_{D}. The binding of N-612-017 or N-612-056 to wildtype or mutant Spike proteins is shown in FIG 36. The binding of N-612-004, N-612-014, N-612-017, and N-612-056 to wildtype Spike or the UK variant Spike protein are shown in FIG 37. Tables 12 and 13 present the K_{D} of the various anti-Spike antibodies against the wildtype and mutant Spike proteins.

| **Table 12 (K_{D} (nM)):** | | | | | | | | | |
|---|---|---|---|---|---|---|---|---|---|
| **RBD Mutation** | **Pango Lineage** | **N-612-017** | **N-612-017-01** | **N-612-017-03** | **N-612-017-05B02** | **N-612-017-05B05** | **N-612-017-5B05 SV** | **N-612-056** | **N-612-056-21** |
| Wildtype | | 5.29 | 0.64 | 0.25 | 0.11 | 3.28 | 6.62 | 2.98 | 0.41 |
| K417N | - | 5.4 | NT | NT | NT | NT | NT | 3.5 | NT |
| N501Y | Alpha | 7.25 | 1.36 | 0.50 | 0.21 | 4.93 | NT | 1.92 | 0.69 |
| K417N/N201Y | - | 7.5 | | | | | | 2.8 | |
| E484K | Zeta/Eta /Iota | 49.7 | 0.37 | 0.21 | 0.18 | 2.68 | NT | 2.52 | 0.62 |
| K417N/E484K | - | 56.5 | | | | | | 3.3 | |
| E484K/N501Y | Theta | 63.1 | 0.57 | 0.21 | 0.12 | 2.38 | NT | 1.92 | 0.45 |
| K417N/E484K /N501Y | Beta | 30.6 | 0.77 | 0.35 | 0.15 | 2.56 | 3.23 | 2.43 | 0.11 |
| L452R | - | 51.2 | NT | NT | NT | NT | NT | 0.45 | NT |
| K417T/E484K /N501Y | Gamma | 3.79 | 0.29 | 0.21 | 0.14 | 1.55 | NT | 1.32 | 0.13 |
| L452R/T478K | Delta | 40.0 | 25.6 | 20.3 | 2.21 | 1.13 | 1.28 | 0.75 | 0.11 |
| L452R | Epsilon | NB | 33.1 | 58.7 | 6.16 | 2.44 | NT | 1.44 | 0.11 |
| L452R/E484Q | Kappa | NB | 13.2 | 12.7 | 1.70 | 0.99 | NT | 0.78 | 0.29 |
| L452Q/F490S | Lambda | NB | NB | NB | Very weak | NB | NB | 5.82 | 0.61 |
| G339D/S371L/ S373P/S375F/ K417N/ N440K/G446S /S447N/T478K /E484A/ Q493R/G496S /Q498R/ N501Y/Y505H | Omicron | 17.4 | | 0.32 | | | 2.18 | | 0.28 |

| | | | | | | | | | |
|---|---|---|---|---|---|---|---|---|---|
| NT: Not tested; NB: No binding | | | | | | | | | |

| **Table 13:** | | | | |
|---|---|---|---|---|
| **Mutation** | **N-612-004 K_{D} (nM)** | **N-612-014 K_{D} (nM)** | **N-612-017 K_{D} (nM)** | **N-612-056 K_{D} (nM)** |
| Wildtype | 0.62 | 11.4 | 12.0 | 4.5 |
| ΔH69/ΔV70/ΔY144/N501Y/ A570D/ D614G/P681H (UK variant) | 2.4 | 14.8 | 15.9 | 4.2 |

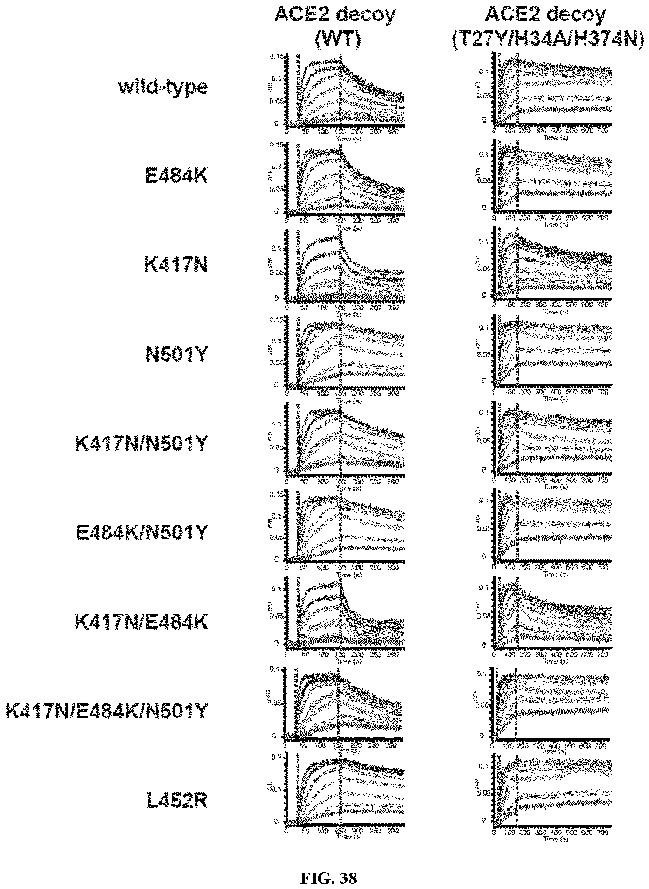

**Example 14:** ACE2 decoy peptides were exposed to wildtype and mutant Spike proteins and the binding affinities of the ACE2 decoy peptides for the Spike proteins were measured. All experiments used a running buffer composed of 10 mMHEPES, pH 7.4, 150mM NaCl, 0.02% tween 20, 0.1% BSA unless indicated otherwise. For 1:1 binding affinity determination of ACE2-decoy peptides against SARS-CoV-2 RBD wild-type and mutants, ACE2-decoy peptides were immobilized onto AHC sensor (Sartorius Corporation), and the RBD (wild-type or mutants) concentration series of 200, 100, 50, 25, 12.5, 6.25, 3.125 nM were used to determine K_{D}. The binding of wildtype ACE2-IgG₁Fc and the T27Y/H34A/H374N triple mutant ACE2 decoy-IgGiFc to wildtype or mutant Spike proteins is shown in FIG 38. Table 14 present the Kₒₙ, K_{off}, and K_{D} of the ACE2 decoy peptides against the wildtype and mutant Spike proteins.

| **Table 14** | **ACE2(WT)-IgG₁Fc** | | | **ACE2(T27Y/H34A/H374N)-IgG₁Fc** | | |
|---|---|---|---|---|---|---|
| **RBD mutants** | **Kₒₙ (1/Ms)** | **K_{off} (1/s)** | **K_{D} (nM)** | **Kₒₙ (1/Ms)** | **K_{off}** | **K_{D} (nM)** |
| Wild-type | 5.25×10⁵ | 4.90×10⁻³ | 9.33 | 6.91×10⁵ | 2.17×10⁻⁴ | 0.315 |
| E484K | 8.00×10⁵ | 7.02×10⁻³ | 8.77 | 1.00×10⁶ | 3.95×10⁻⁴ | 0.393 |
| K417N | 3.97×10⁵ | 1.35×10⁻² | 34.0 | 4.46×10⁵ | 9.33×10⁻⁴ | 2.090 |
| K417N/E484K | 4.84×10⁵ | 1.73×10⁻² | 35.7 | 8.03×10⁵ | 1.10×10⁻³ | 1.370 |
| N501Y | 6.38×10⁵ | 1.45×10⁻³ | 2.27 | 9.50×10⁵ | 1.40×10⁻⁴ | 0.148 |
| E484K/N501Y | 8.26×10⁵ | 1.76×10⁻³ | 2.13 | 1.22×10⁶ | 1.16×10⁻⁴ | 0.095 |
| K417N/N501Y | 4.57×10⁵ | 3.47×10⁻³ | 7.60 | 7.47×10⁵ | 3.34×10⁻⁴ | 0.447 |
| K417N/E484K/ N501Y | 7.45×10⁵ | 3.93×10⁻³ | 5.28 | 7.56×10⁵ | 3.51×10⁻⁴ | 0.465 |
| L452R | 4.39×10⁵ | 1.19×10⁻³ | 2.71 | 4.62×10⁵ | 1.02×10⁻⁴ | 0.221 |

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Particular embodiments of this invention are described herein, including the best mode known to the inventors for carrying out the invention. Variations of those particular embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An antigen binding fragment (Fab fragment) comprising a heavy variable (V_{H}) chain having at least 85% sequence identity to each of SEQ ID NOs: 127, 128, and 130, and one of SEQ ID NOs: 129, 243, and 245 and a light variable (V_{L}) chain having at least 85% identity to each of SEQ ID NOs: 131 & 132, wherein each of the V_{H} chain and the V_{L} chain have three complementarity determining regions (CDRs), and wherein
the V_{H} CDRs comprise SEQ ID NOs: 67-69 and the V_{L} CDRs comprise SEQ ID NO: 70;
the V_{H} CDRs comprise SEQ ID NOs: 67, 68, and 241 and the V_{L} CDRs comprise SEQ ID NO: 70;
the V_{H} CDRs comprise SEQ ID NOs: 63, 240, and 69 and the V_{L} CDRs comprise SEQ ID NO: 70;
the V_{H} CDRs comprise SEQ ID NOs: 238, 68, and 242 and the V_{L} CDRs comprise SEQ ID NO: 70;
the V_{H} CDRs comprise SEQ ID NOs: 239, 68, and 242 and the V_{L} CDRs comprise SEQ ID NO: 70; or
the V_{H} CDRs comprise SEQ ID NOs: 43, 44, 127-129, and 243 and the V_{L} CDRs comprise SEQ ID NO: 46.

2. The Fab fragment of claim 1, wherein the V_{H} CDRs comprise SEQ ID NOs: 67-69, 127, 128, 243, and 130 and the V_{L} CDRs comprise SEQ ID NO: 70.

3. The Fab fragment of claim 1, wherein the V_{H} CDRs comprise SEQ ID NOs: 67, 68, 127, 128, 130, 243, and 241 and the V_{L} CDRs comprise SEQ ID NO: 70.

4. The Fab fragment of claim 1, wherein the V_{H} CDRs comprise SEQ ID NOs: 63, 69, 127, 128, 130, 240, and 243 and the V_{L} CDRs comprise SEQ ID NO: 70.

5. The Fab fragment of claim 1, wherein the V_{H} CDRs comprise SEQ ID NOs: 68, 127, 128, 130, 238, 242, and 243 and the V_{L} CDRs comprise SEQ ID NO: 70.

6. The Fab fragment of claim 1, wherein the V_{H} CDRs comprise SEQ ID NOs: 68, 127, 128, 130, 239, 242, and 245 and the V_{L} CDRs comprise SEQ ID NO: 70.

7. The Fab fragment of claim 1, wherein the V_{H} CDRs comprise SEQ ID NOs: 43, 44, 127-130, 128, 243, and 130 and the V_{L} CDRs comprise SEQ ID NO: 46.

8. The Fab fragment of any one of claims 1-7, wherein the V_{H} chain has an arrangement (SEQ ID NO:127)-V_{H} CDR1-(SEQ ID NO:128)-V_{H} CDR2-(SEQ ID NO:129)-V_{H} CDR3-(SEQ ID NO:130).

9. The Fab fragment of any one of claims 1-7, wherein the V_{H} chain has an arrangement (SEQ ID NO:127)-V_{H} CDR1-(SEQ ID NO:128)-V_{H} CDR2-(SEQ ID NO:243)V_{H} CDR3-(SEQ ID NO:130).

10. The Fab fragment of any one of claims 1-9, wherein the V_{L} has an arrangement (SEQ ID NO:131)-V_{L} CDR1-(SEQ ID NO:132).

11. The Fab fragment of any one of claims 1-10, wherein the Fab fragment further comprises a crystallizable fragment (Fc) domain.

12. The Fab fragment of any one of claims 1-11, wherein the Fab fragment is bound to a severe acute respiratory system type 2 coronavirus (SARS-CoV-2) spike protein.

13. The Fab fragment of claim 13, wherein the spike protein has one or more point mutations selected from the group consisting of S131, L18F, T19R, T20N, P26S, A67V, ΔH69, ΔV70, G75V, T76I, D80A, T95I, D138Y, G142D, ΔL141, ΔG142, ΔV143, ΔY144, ΔY145, W152C, E154K, E156G, ΔF157, ΔR158, R190S, N211I, ΔL212, R214ins, D215G, ΔL241, ΔL242, L242H, ΔA243, ΔL244, R246N, ΔS247, ΔY248, ΔL249, ΔT250, ΔP251, ΔG252, ΔD253, G339D, S371L, S373P, S375F, K417N, K417T, N440K, G446S, S447N, L452Q, L452R, T478K, E484A, E484K, E484Q, F490S, Q493R, G496S, Q498R, N501Y, Y505H, T547K, A570D, D614G, H655Y, N679K, P681H, P681R, A701V, T716I, D796Y, N858K, T859N, D950N, Q954H, S982A, N969K, L981F, Q1071H, E1095K, H1101Y, D1118H, T10271, V1176F, and a combination thereof.

14. A polynucleotide encoding the Fab fragment of any one of claims 1-13.

15. The Fab fragment of any one of claims 1-13 for use as a medicament, such as for use in preventing or treating COVID-19 in a subject in need thereof.
